# EUROPEAN PATENT APPLICATION

(11) **EP 1 486 212 A1**
(43) Date of publication of application: **15.12.2004**
(21) Application number: 04018827.8
(22) Date of filing: 01.06.2001
(51) Int. Cl.: A61K 35/76

(54) **Combination of a mutant herepes virus and irinotecan for the treatment of cancer**

(30) Priority: 01.06.2000 US 208546 P
(62) Divisional of application: 01946051.8
(71) Applicant: Sloan-Kettering Institute For Cancer Research, New York, New York 10021 (US)
(72) Inventor: Fong, Yuman, New York, NY 10021 (US); Bennett, Joseph, New York, NY 10021 (US); Petrowsky, Henrik, 61130 Nidderau (DE)
(74) Representative: Bösl, Raphael, Dr.rer.nat., Dipl.-Chem.

(57) **Abstract**

This invention provides methods of treating cancer employing attenuated mutant herpes viruses and irinotecan.

## Description

### Field of the Invention

This invention relates to methods of treating cancer.

### Background of the Invention

G207 is a ribonucleotide reductase-negative herpes simplex virus (HSV) type 1, which was designed for brain tumor therapy and is currently under clinical evaluation as new treatment for malignant glioma (Markert et al., Gene Ther. 7:867-874, 2000; Mineta et al., Nature Med. 1:983-943, 1995). Recently, this HSV mutant was shown to also demonstrate high oncolytic potency against colorectal cancer cells (Kooby et al., FASEB J. 13:1325-1334, 1999). Recently, this virus has also been found to be effective in experimental treatments of many types of cancers, including breast cancer (Wu et al., Cancer Research 61(7):3009-3015, 2001), prostate cancer (Oyama et al., Japanese Journal of Cancer Research 91(12):1339-1344, 2000), colorectal cancer (Delman et al., Human Gene Therapy 11(18):2465-2472, 2000), gastric cancer (Journal of Molecular Medicine 78(3):166-174, 2000), bladder cancer (Oyama et al., Human Gene Therapy 11(12):1683-1693, 2000), ovarian cancer (Coukos et al., Cancer Gene Therapy 7(2):275-283, 2000), head and neck cancer (Carew et al., Human Gene Therapy 10(10):1599-1606, 1999), and pancreatic cancer (Lee et al., Journal of Gastrointestinal Surgery 3(2):127-131, 1999).

G207 typifies the strategy used in many candidate oncolytic viruses that specifically target tumor cells by deletion of viral ribonucleotide reductase (RR) and *γ*_{*1*}*34*.*5*. First, viral RR is inactivated by inserting the *Escherichia coli lacZ* gene into the *infected cell protein 6 (ICP6)* locus that codes for the large subunit of RR. RR catalyzes the reduction of ribonucleotides to the corresponding deoxyribonucleotides, thereby providing sufficient precursors for the *de novo* synthesis of DNA. In mammalian cells, RR is highly expressed during S-phase and under DNA damage/repair conditions (Björklund et al., Biochemistry 29:2452-5458, 1990; Chabes et al., J. Biol. Chem. 275:17747-17753, 2000; Engstöm et al., J. Biol. Chem. 260:9114-9116, 1985; Filatov et al., J. Biol. Chem. 270:25239-25243, 1995; Tanaka et al., Nature 404:42-49, 2000). Most herpes viruses encode their own RR, and their replication is, therefore, independent of the host cell cycle (Boehmer et al., Annu. Rev. Biochem. 66:347-384, 1997; Roizman et al., Fields Virology, 3^{rd} ed. Lippincott-Raven, Philadelphia 1996; Roizman et al., Fields Virology, 3^{rd} ed. Lippincott-Raven, Philadelphia, 1996). The inactivation of ICP6 in G207 makes viral DNA replication completely dependent on the cellular enzyme and, consequently, replication of this mutant becomes largely dependent on host cell conditions. It is, therefore, reasonable to conceive that cell cycle alterations or DNA damage/repair conditions might modulate the replication of this herpes vector. The second mutation in G207 is the deletion of both *γ*_{*1*}*34.5* loci. The *γ*_{*1*}*34.5* gene codes a protein (ICP34.5) with at least two functions. One allows HSV to replicate and spread within central nervous system (Chou et al., Science 250:1262-1266, 1990; Whitley et al., J. Clin. Invest. 91:2837-2843, 1993). The second function confers HSV with the ability to escape from a host defense mechanism against viral infections by preventing the cellular shut-off of protein synthesis (Chou et al., Proc. Natl. Acad. Sci. U.S.A. 92:10516-10520, 1995; He et al., Proc. Natl. Acad. Sci. U.S.A. 94:843-848, 1997). This function can be substituted by the ICP34.5 homologous domain of the cellular growth arrest and DNA damage protein 34 (GADD34), which is a protein that is induced by DNA damage (He et al., Proc. Natl. Acad. Sci. U.S.A. 94:843-848, 1997).

Chemotherapy is an established modality in the treatment of malignancies. Fluorodeoxyuridine (FUdR) is a widely used chemotherapeutic drug to treat colorectal cancer. It is rapidly converted to the active metabolite 2'-deoxy-5-fluorouridine 5' monophosphate (FdUMP) by phosphorylation via thymidine kinase. FdUMP inhibits the enzyme thymidylate synthetase (TS) by forming a covalent complex with both sulfhydryl residue of TS and methylenetetrahydrofolate. Inhibition of TS causes a depletion of deoxythymidine 5' triphosphate (dTTP) with subsequent imbalance of intracellular deoxynucleotide triphosphate pools (Daneberg et al., Mol. Cell Biochem. 43:49-57, 1982; Jackson, J. Biol. Chem. 253:7440-7446, 1978; Yoshioka et al., J. Biol. Chem. 262:8235-8241, 1987). This inhibition induces cytotoxicity through several mechanisms. Nucleotide pool imbalances have been shown to induce a specific endonuclease with double-strand breakage activity in FM3A cells (Yoshioka et al., J. Biol. Chem. 262:8235-8241, 1987). Other studies have demonstrated that excessive dUTP/dTTP ratios result in uracil misincorporation and misrepair leading to DNA strand breaks (Ayusawa et al., J. Biol. Chem. 258:12448-12454, 1983; Goulian et al., Adv. Exp. Med. Biol. 195:89-95, 1986; Ingraham et al., Biochemistry 25:3225-3230, 1986). The ability of FUdR to be incorporated into nascent DNA has been suggested as another mechanism of cytotoxic action (Danenberg et al., Biochem. Biophys. Res. Commun. 102:654-658, 1981). Furthermore, FUdR has profound effects on cell cycle and DNA replication by causing early S-phase blockade, loss of histone H1, and retarded DNA elongation (D'Anna et al., Biochemistry 24:5020-5026, 1985).

FUdR and other thymidylate synthase inhibitors are examples of chemotherapeutic agents that act by disrupting the balance of nucleotide production in cells. Additional agents have similar effects, including pyrimidine analogs, purine analogs, methotrexate, and 5-FU hydroxyurea. Another type of chemotherapeutic agent, the antimetabolites, acts by interferring with DNA synthesis. Alkylating agents, some anticancer antibiotics, and intercalating agents act by direct interaction with DNA, and can lead to, for example, disruption in DNA synthesis and/or transcription, and possibly lead to DNA breakage. Mitomycin C (MMC) is an antitumor antibiotic, has a wide clinical spectrum of antitumor activity, and is standard therapy for gastric cancer (Kelsen, Seminars in Oncology 23:379-389, 1996). MMC binds DNA by mono- or bifunctional alkylation, leading to DNA strand cross-linking and inhibition of DNA synthesis (Verweij et al., Anti-Cancer Drugs 1:5-13, 1990).

### Summary of the Invention

The invention provides methods of treating cancer in patients. These methods involve administration of (i) an attenuated herpes virus in which a γ34.5 gene (or genes) and/or a ribonucleotide reductase gene is inactivated, and (ii) a chemotherapeutic drug to patients. An example of an attenuated herpes virus that can be used in these methods is G207. The chemotherapeutic drug can be, e.g., an alkylating agent, such as busulfan, caroplatin, carmustine, chlorambucil, cisplatin, cyclophosphamide, dacarbazine, ifosfamide, lomustine, mecholarethamine, melphalan, procarbazine, streptozocin, or thiotepa; an antineoplastic antibiotic, such as bleomycin, dactinomycin, daunorubicin, doxorubicin, idarubicin, mitomycin (e.g., mitomycin C), mitoxantrone, pentostatin, or plicamycin; an antimetabolite, such as a thymidylate synthetase inhibitor (e.g., fluorodeoxyuridine), cladribine, cytarabine, floxuridine, fludarabine, flurouracil, gemcitabine, hydroxyurea, mercaptopurine, methotrexate, or thioguanine.

Cancers that can be treated using the methods of the invention include, for example, astrocytoma, oligodendroglioma, meningioma, neurofibroma, glioblastoma, ependymoma, Schwannoma, neurofibrosarcoma, neuroblastoma, pituitary adenoma, medulloblastoma, head and neck cancer, melanoma, prostate carcinoma, renal cell carcinoma, pancreatic cancer, breast cancer, lung cancer, colon cancer, gastric cancer, bladder cancer, liver cancer, bone cancer, fibrosarcoma, squamous cell carcinoma, neurectodermal, thyroid tumor, Hodgkin's lymphoma, non-Hodgkins lymphoma, hepatoma, mesothelioma, epidermoid carcinoma, and cancers of the blood. The viruses used in the methods of the invention can also include a gene encoding a heterologous gene product, such as a vaccine antigen or an immunomodulatory protein.

The invention further includes the use of the viruses and anticancer compounds described herein in the treatment of cancer, and the use of these agents in the preparation of medicaments for treating cancer. For example, the invention includes the use of the viruses described herein in the preparation of a medicament for administration to a patient in conjunction with an anticancer compound as described herein, as well as the use of such an anticancer compound in the preparation of a medicament for administration to a patient in conjunction with a virus as described herein.

The invention provides several advantages. For example, as is discussed further below, the therapeutic agents used in the invention, mutant Herpes viruses and anticancer agents, have synergistic activities in the treatment of cancer. As a result of this synergism, a dose-reduction for each agent can be accomplished over a wide range of drug-effect levels, without sacrificing therapeutic efficacy. Using lower amounts of the agents has several benefits, including minimization of toxicity to treated patients, as well as decreased costs. An additional advantage of the methods of the invention is that medical professionals are very familiar with the use of many of the anticancer agents that are used in the invention. For instance, the toxicities of many of the agents used in the invention are well recognized, and therapies exist to treat any associated side effects. In addition, mutant herpes viruses that can be used in the invention replicate in, and thus destroy, dividing cells, such as cancer cells, while not affecting other, quiescent cells in the body. These herpes viruses can also be multiply mutated, thus eliminating the possibility of reversion to wild type. Moreover, if necessary, the replication of herpes viruses can be controlled through the action of antiviral drugs, such as acyclovir, which block viral replication, thus providing another important safeguard. Finally, in some examples of the methods of the invention, anticancer agents, such as mitomycin C, are used to counteract the decreased replication phenotype γ34.5 gene deletions of certain herpes virus vectors, without the potential risk of increasing neurovirulence.

Other features and advantages of the invention will be apparent from the following detailed description, the drawings, and the claims.

### Brief Description of the Drawings

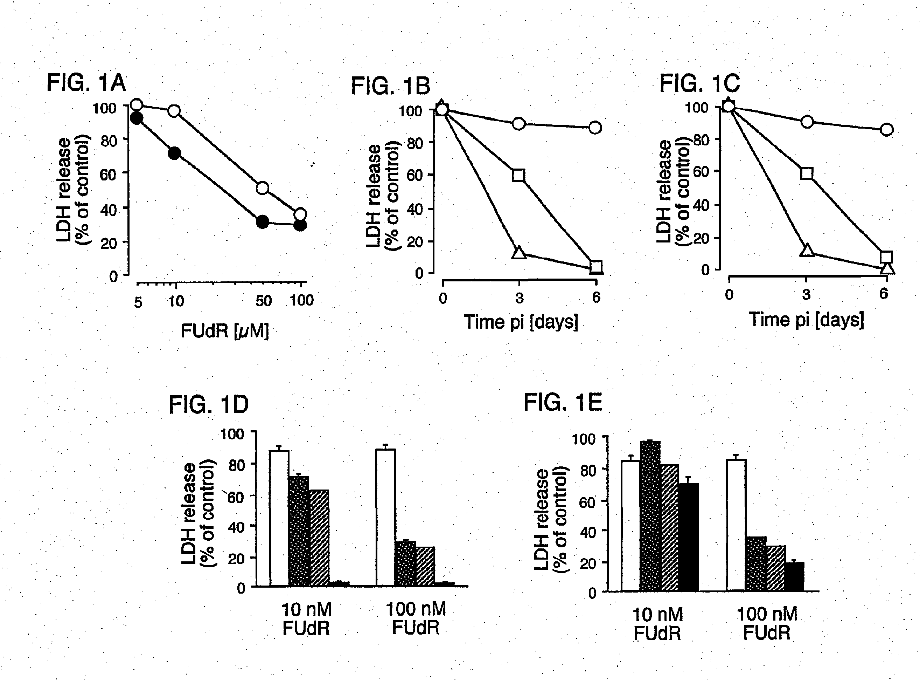

**Figure 1** depicts the results of experiments showing the cytotoxic effects of G207 and FUdR. Cell viability was assessed as function of maximal release of intracellular LDH. *Upper figure panel,* cytotoxicity of G207 and FUdR. HCT8 (*black circle*) and HCT8/7dR (*white circle*) were exposed to cumulative FUdR concentrations (5, 10, 50, 100 nM) and viability was determined at day 6 following start of treatment (*A*). Viral cytotoxicity in HCT8 (*B*) and HCT8/7dR (*C*) at day 3 and 6 pi. Cells were infected with G207 at an MOI of 1.0 (Δ), 0.1 (□), and 0.01 (○). *Lower figure panel,* cell viability for single (G207 or FUdR) and combined treatment in HCT8 (*D*) and HCT8/7dR (*E*) at day 6. Cells were infected with G207 at an MOI of 0.01 (*white bar*), exposed to FUdR (*gray bar*), or treated with G207 (MOI = 0.01) and FUdR (10 nM or 100 nM) in combination (*black bar*). Additive effects were calculated as product from each single treatment (*oblique bar*). All assays were performed in quadruplicate for each condition (avg ± SEM).

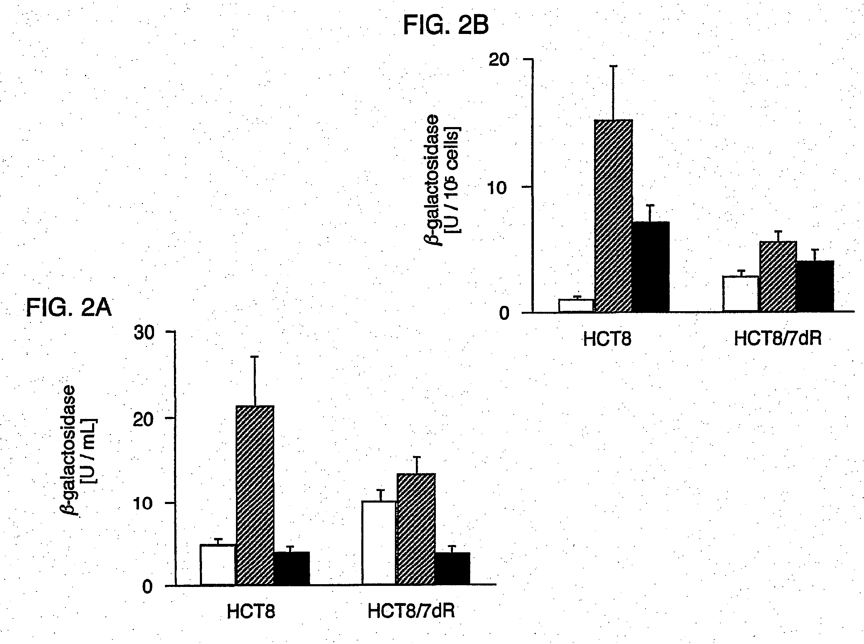

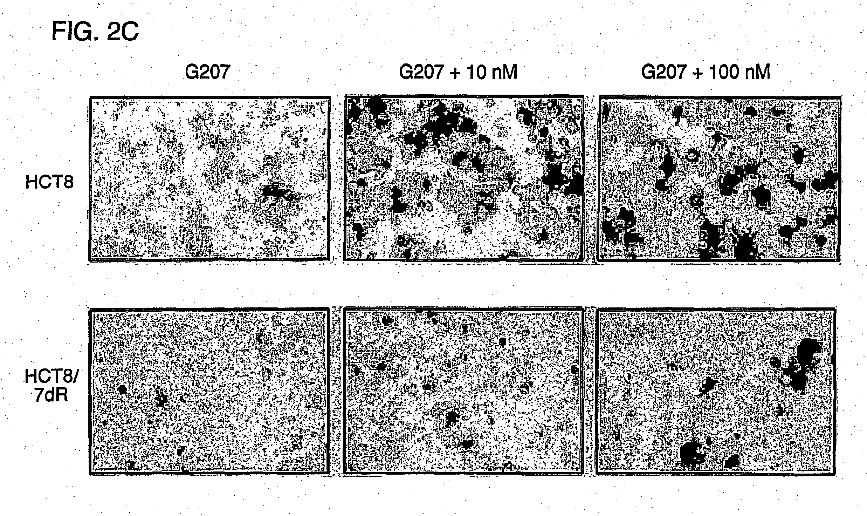

**Figure 2** depicts the results of experiments showing the influence of FUdR on β-galactosidase expression following infection with G207. Cells (2 x 10⁴) were plated in 24-well plates, and were infected with G207 at an MOI of 0.01 in presence (10 nM, *oblique*; 100 nM, *black*) and absence (*white*) of FUdR. At day 3 following infection, cells were lysed and total β-galactosidase activity of cell lysates was measured (A). Cell counts for each condition were determined in additional wells by trypan blue exclusion and specific activity was calculated by referring total activity to viable cell number (B). All assays were performed in triplicates (avg ± SEM). For *lacZ* staining 3 × 10⁵ cells were plated onto 25 cm² flasks. Twelve hours later cells were infected with G207 in the presence and absence of FUdR under the same conditions as described above. At day 3 pi, cytospin slides were prepared and stained with X-gal for *lacZ* expression. Figure 2, *panel C* shows representative fields at 200-fold magnification for different treatment conditions.

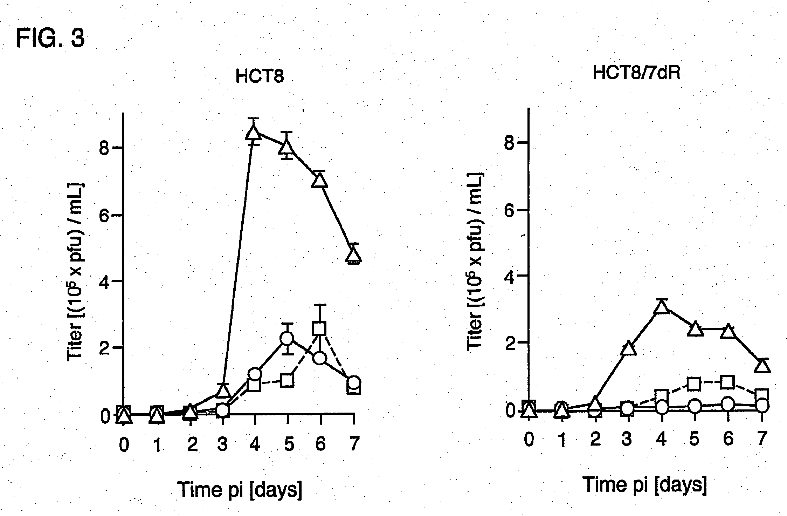

**Figure 3** depicts the results of experiments showing the effect of FUdR on viral replication. Viral titers were determined to evaluate the influence of FUdR on viral replication. 5 x 10⁴ cells were plated per well in 12-well plates. Twelve hours later, cells were infected at an MOI of 0.01 in presence (Δ 10 nM; ○ 100 nM) and absence (□ control) of FUdR. Supernatants and cells were harvested daily for following 7 days pi and lysates were titrated on Vero cells by standard plaque assay. All assays were performed in triplicates for each condition (avg ± SEM).

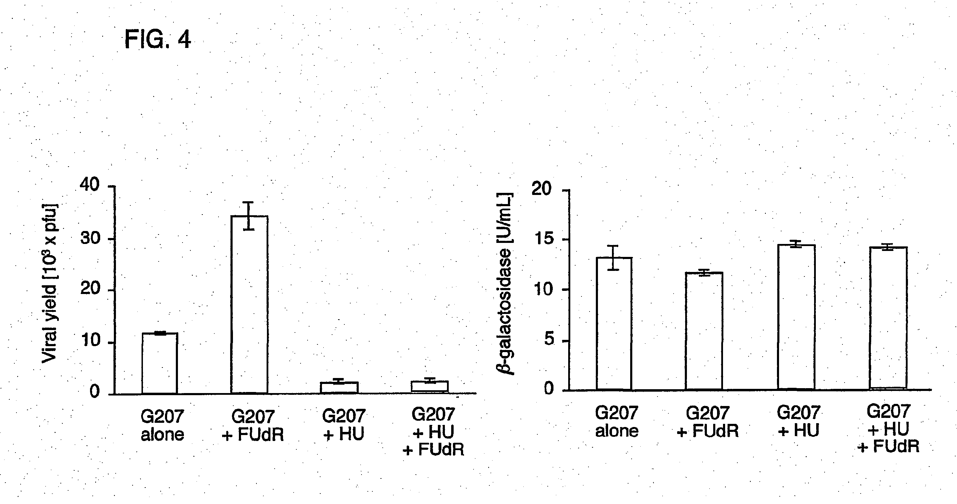

**Figure 4** depicts the results of experiments showing the effects of FUdR and HU on viral replication. HCT8 cells were infected with 2 pfu of G207 per cell. After adsorption of 1 hour at 37°C inoculum was removed, cells were washed with PBS, and medium containing 10 nM FUdR or control medium without FUdR was added. At 8 hours pi, infected cells in presence and absence of FUdR were exposed to 1 mM HU. At 36 hours pi cells and supernatant were harvested and lysates were prepared by three cycles of freezing and thawing. Viral titers (*A*) and β-galactosidase activity (*B*) of the lysate were determined. All assays were performed in triplicates for each condition (avg ± SEM).

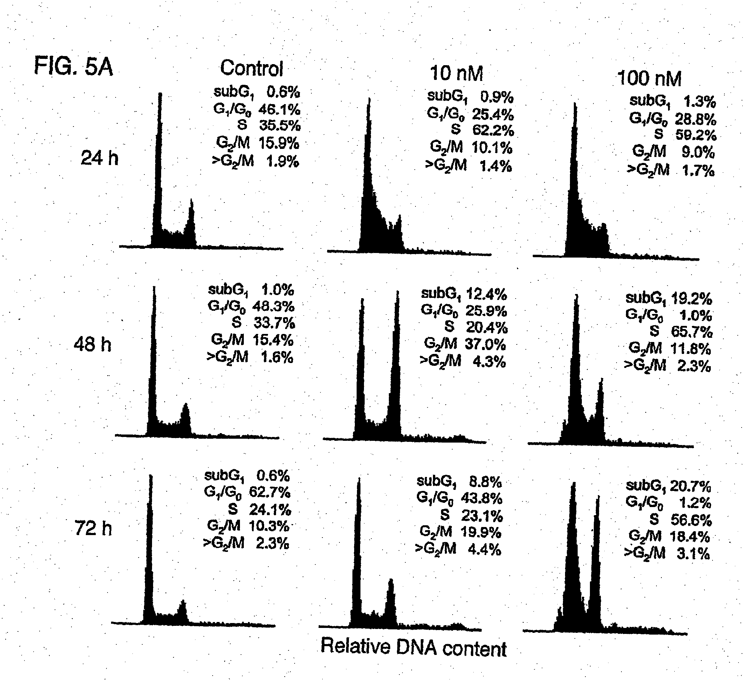

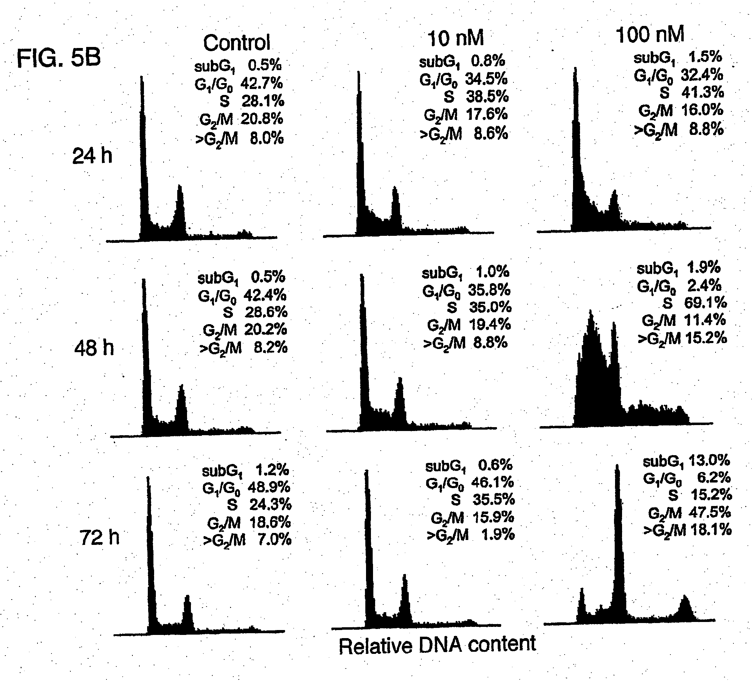

**Figure 5** depicts the results of experiments showing the effect of FUdR on the cell cycle. Asynchronously growing cells (1 × 10⁶) were plated onto 75 cm² in 20 ml of media. Twelve hours later FUdR was added to media to a final concentration of 10 nM and 100 nM. Untreated cells served as control. DNA content was measured on ethidium bromide-stained nuclei by FACS analysis at 24 h, 48 h, and 72 h following start of treatment. Cell cycle analysis of HCT8 (A) and HCTB/7dR (B) was performed based on the shown side scatter histograms. Histograms were gated for subG₁ fraction (DNA < G₁/G₀) and DNA > G₂/M.

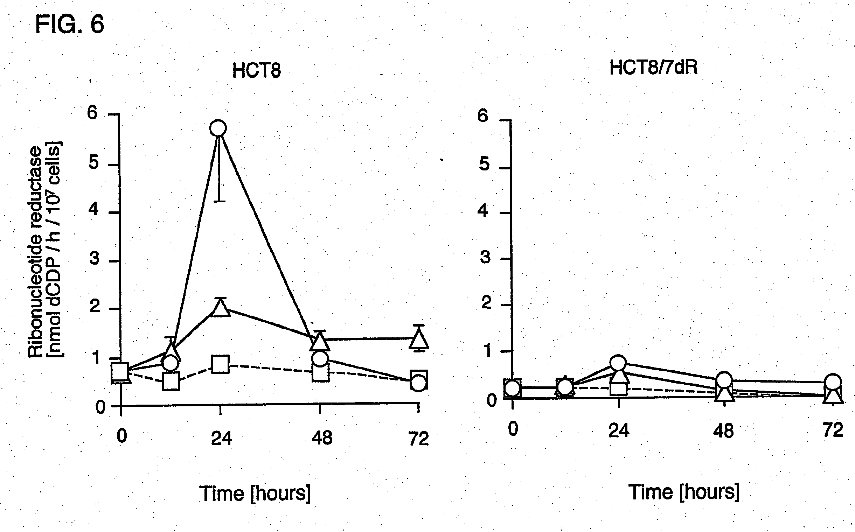

**Figure 6** depicts the results of experiments showing the effect of FUdR on cellular ribonucleotide reductase activity. 1 × 10⁷ cells were plated onto 225 cm² flasks. After 9 hours FUdR was added to the media to a final concentration of 10 nM and 100 nM. Untreated cells served as control. Ribonucleotide reductase activity was measured in cellular extracts at various time points in presence (Δ 10 nM; ○ 100 nM) and absence (□ control) of FUdR. Activities were referred to cell count. All assays were performed in triplicate for each time point and condition (avg ± SEM).

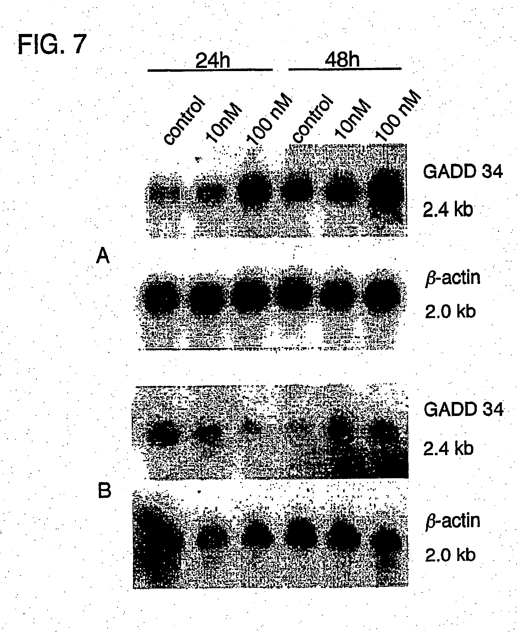

**Figure 7** depicts the results of experiments showing GADD34 expression in response to FUdR. Northern blots of GADD34 mRNA in HCT8 (A) and HCT8/7dR (B) cells grown in absence and presence of 10 nM and 100 nM FUdR for 24 and 48 hours. Cells were plated and treated with FUdR according to the experiment of RR measurement (see legend to Figure 5). β-actin served as loading controls for GADD34.

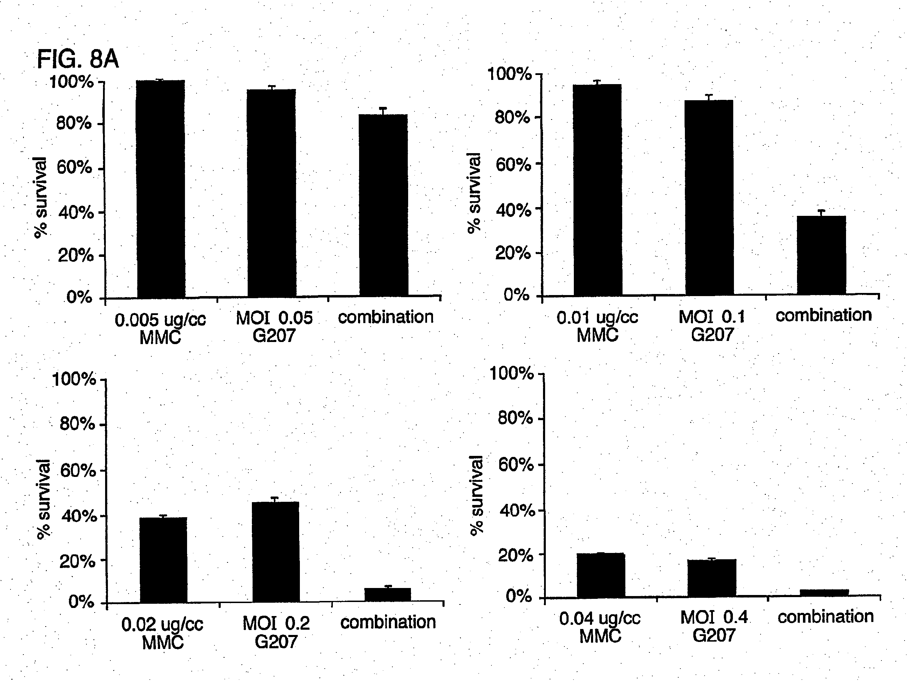

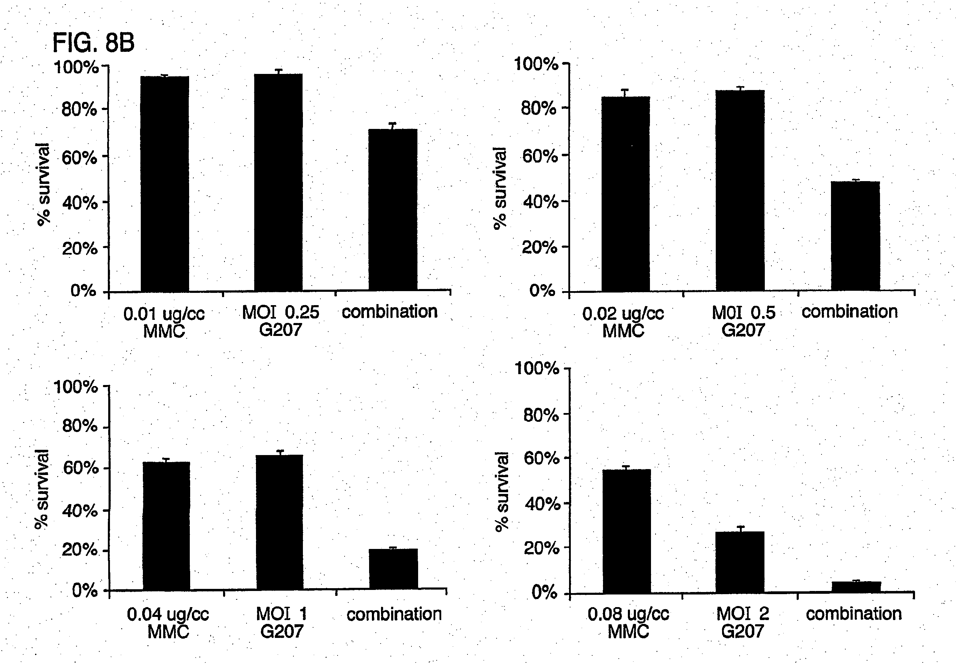

**Figure 8** depicts the results of experiments showing that combination chemotherapy and oncolytic viral therapy to kill gastric cancer cells demonstrates enhanced efficacy as compared to single agent therapy alone. OCUM-2MD3 (A) MKN-45-P (B) gastric cancer cells were treated with different doses of Mitomycin C (µg/cc) or G207 (MOI). Combination therapy was performed to keep the ratio of MMC:G207 constant at 1:10 for the OCUM-2MD3 cells, and 1:25 for the MKN-45-P cells. Standard MTT assay was used to assess cytotoxicity for each treatment group with results presented as % survival as compared to control.

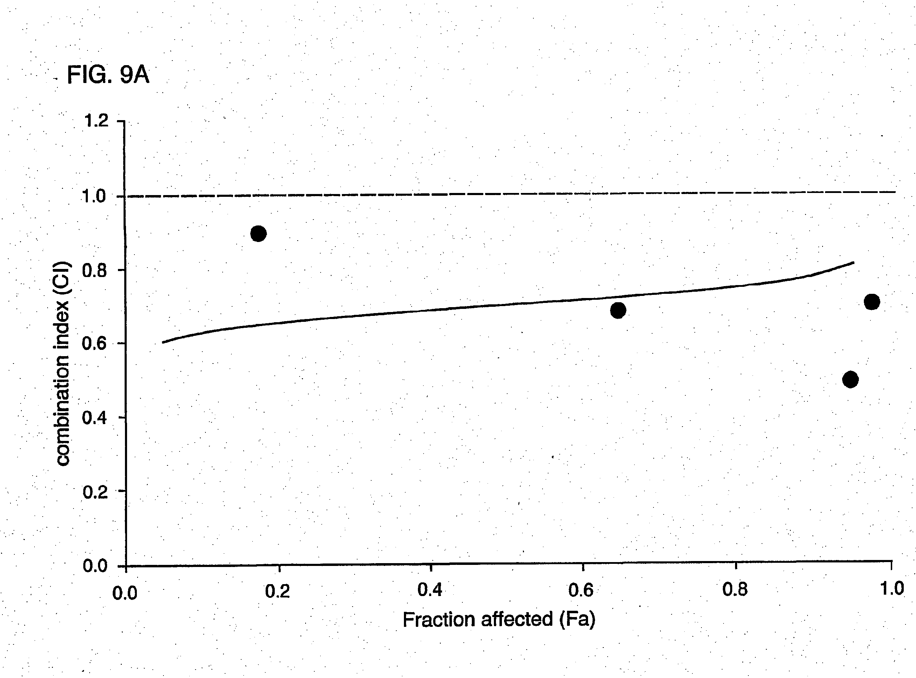

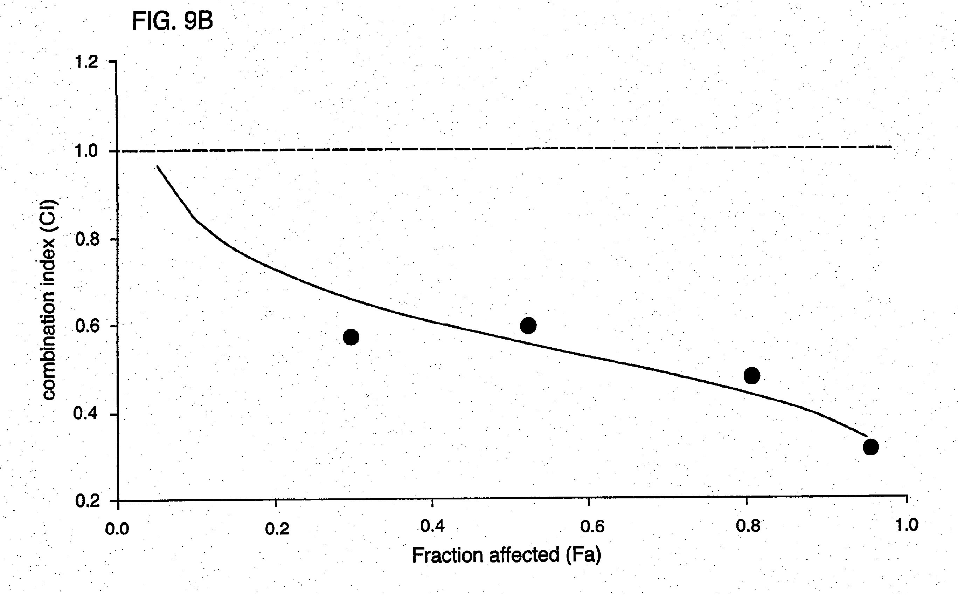

**Figure 9** depicts the results of experiments showing that combination therapy using Mitomycin C and G207 demonstrates a synergistic interaction over the entire range of doses evaluated. The Chou-Talaley combination index method of evaluating synergy was performed as described in Methods (see below). The CI-Fa plot was constructed using experimental data points (dark circles) and by determining CI values over the entire range of Fa values from 5-95% (solid line) using CalcuSyn software. The additive effect of G207 and MMC is represented at CI=1 (dotted line). G207 and MMC combination therapy results in moderate synergy for the OCUM-2MD3 cell line (A) and strong synergy for the MKN-45-P cell line (B) at all effect levels.

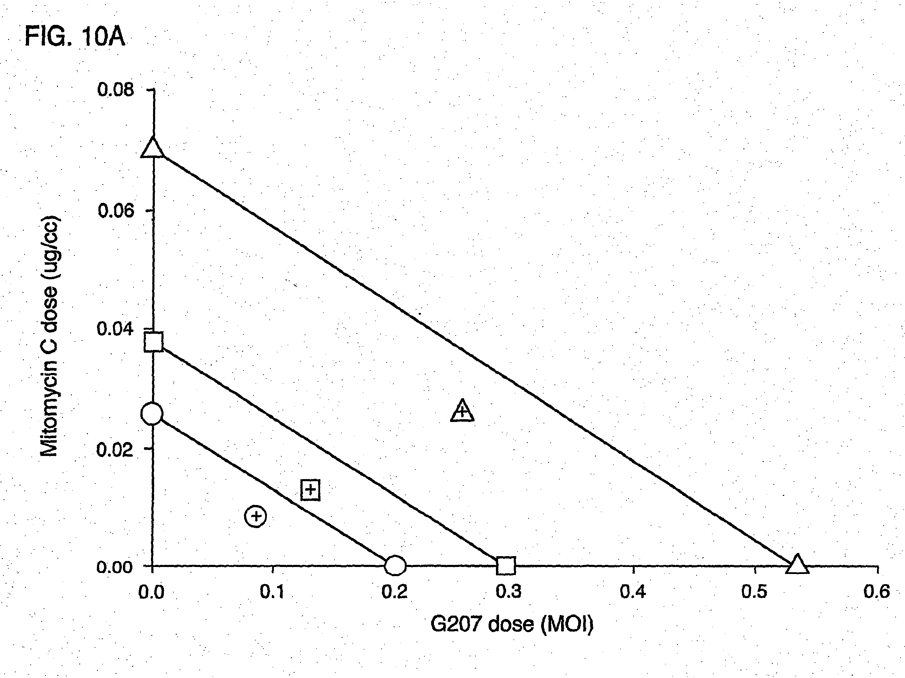

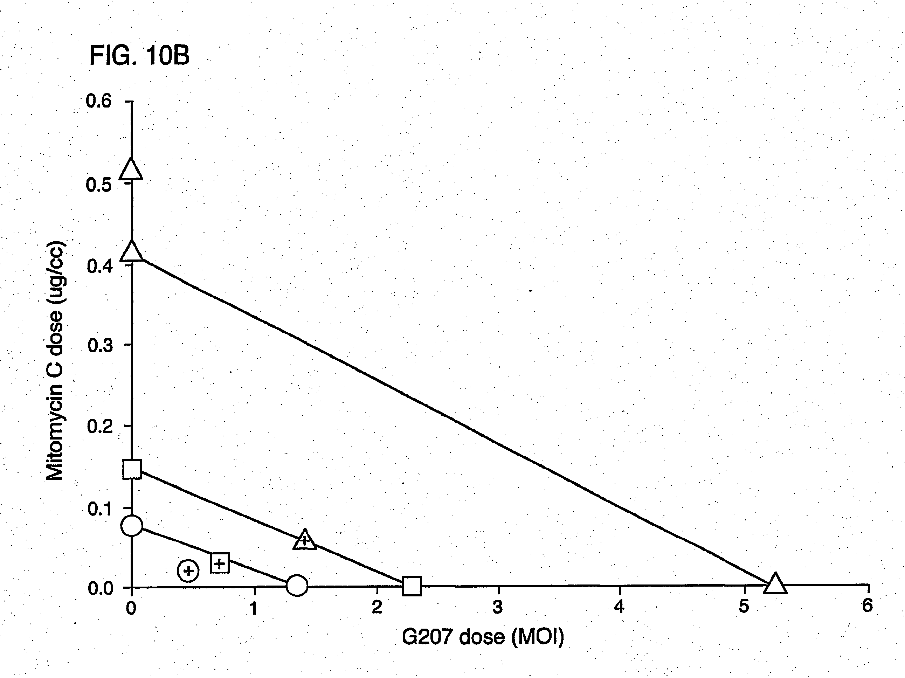

**Figure 10** depicts isobolograms that demonstrate synergism and dose-reduction with G207 and MMC combination therapy in both the OCUM-2MD3 cell line (A) and the MKN-45-P (B) cell line. The doses of MMC and G207 necessary to achieve 90% cell kill (open triangles), 70% cell kill (open squares) and 50% cell kill (open circles) are plotted on the axes, and the connecting solid lines represent the expected additive effects for combination therapy. Experimental combination therapy doses necessary to generate Fa values of 90% (dotted triangles), 70% (dotted squares) and 50% (dotted circles) all lie to the lower left of the corresponding lines, indicating synergism. A dose-reduction using combination therapy is also apparent at all three Fa values for both cell lines.

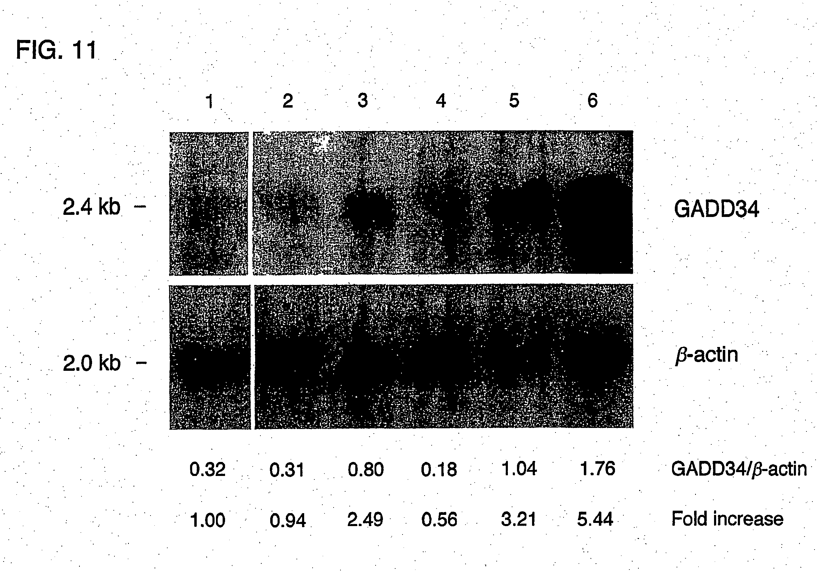

**Figure 11** depicts the results of experiments showing the levels of GADD34 mRNA in OCUM cells exposed to MMC. mRNA extracted from untreated OCUM cells served as the negative control for GADD34 (lane 1), while the positive control (lane 6) demonstrates a strong band at 2.4 kb, the size for GADD34 mRNA. OCUM cells were treated for 24 and 48 hours with either low (0.005 µg/ml) or high dose MMC (0.04 µg/ml). At 24 h, low dose therapy did not result in upregulation of GADD34 mRNA (lane 2), while high dose therapy resulted in a 2.49-fold increase in mRNA as compared to the negative control (lane 3). At 48 h, low dose therapy failed to demonstrate the presence of GADD34 mRNA (lane 4), while high dose therapy resulted in a 3.21-fold increase in mRNA (lane 5).

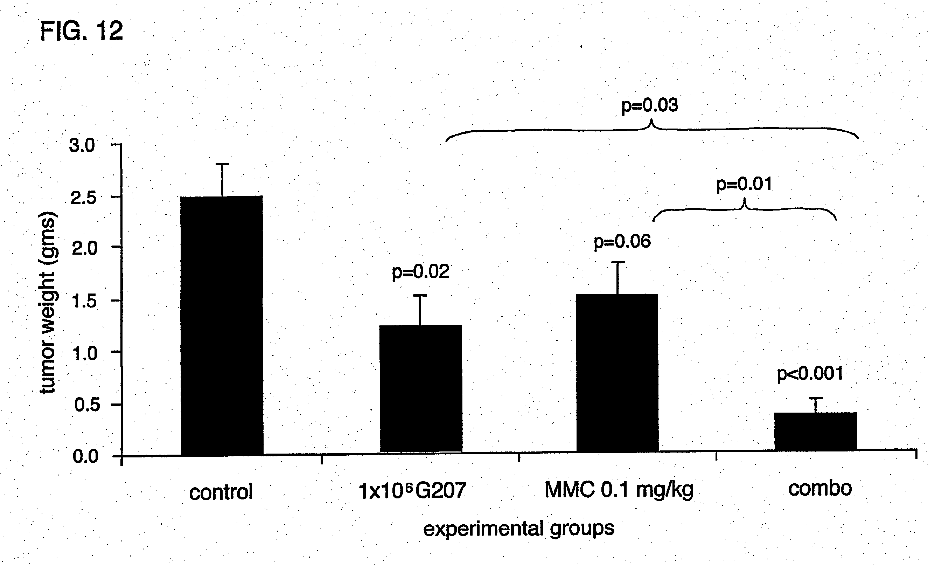

**Figure 12** depicts the results of experiments showing that intraperitoneal chemotherapy and viral therapy demonstrate enhanced tumor kill when given in combination for gastric carcinomatosis. Tumor burden was generated by i.p. injection of OCUM-2MD3 cells into athymic mice and treatment was initiated 3d later. Mice were injected i.p. with media (controls), 1x10⁶ pfu of G207, 0.1 µg/kg MMC, or both agents given in combination. Tumor burden was assessed by weight at 4 weeks post-tumor cell inoculation. There was a significant reduction in tumor burden when comparing G207 therapy to controls (P=0.02), while MMC therapy only demonstrated a trend in tumor burden reduction (vs. controls) at this dose (P=0.06). Combination therapy resulted in the highest reduction in tumor burden when compared to controls (P<0.001), and also showed a significant reduction when compared to G207 therapy (P=0.03) or MMC therapy (P=0.01) alone. Statistical analysis was performed using a two-tailed, Students t-test.

### Detailed Description

The invention provides methods of treating cancer that involve administration of mutant herpes viruses in conjunction with anticancer agents. As is discussed further below, such a combined approach can lead to synergistic effects in the treatment of cancer, thus providing substantial therapeutic benefits (e.g., administration of decreased amounts of potentially toxic chemotherapeutic agents, without loss of therapeutic effect). Examples of mutant herpes viruses and anticancer agents.that can be used in the invention, as well as modes of their administration, are provided below. Also provided below are examples of cancers that can be treated using the methods of the invention, as well as experimental results showing the efficacy of these methods.

### Mutant Herpes Viruses

Mutant viruses that can be used in the invention can be derived from members of the family Herpesviridae (e.g., HSV-1, HSV-2, VZV, CMV, EBV, HHV-6, HHV-7, and HHV-8). Specific examples of attenuated HSV mutants that can be used in the invention include G207 (Yazaki et al., Cancer Res. 55(21):4752-4756, 1995), HF (ATCC VR-260), MacIntyre (ATCC VR-539), MP (ATCC VR-735); HSV-2 strains G (ATCC VR-724) and MS (ATCC VR-540), as well as mutants having mutations in one or more of the following genes: the immediate early genes ICP0, ICP22, and ICP47 (U.S. Patent No. 5,658,724); the y34.5 gene; the ribonucleotide reductase gene; and the VP16 gene (i.e., Vmw65, WO 91/02788; WO 96/04395; WO 96/04394). The vectors described in U.S. Patent Nos. 6,106,826 and 6,139,834 can also be used.

As is discussed further below, a preferred mutant herpes virus for use in the methods of the invention has an inactivating mutation, deletion, or insertion in one or both γ34.5 genes and/or a ribonucleotide reductase gene. One example of such a mutant herpes virus is G207, which, as is described above, has deletions in both copies of the γ34.5 gene, which encodes the major determinant of HSV neurovirulence. G207 also includes an inactivating insertion in *UL39*, which is the gene encoding infected-cell protein 6 (ICP6), the large subunit of ribonucleotide reductase of this virus.

An additional examples of a Herpes virus mutant that can be used in the invention is G47Δ, which is a multimutated, replication-competent HSV-1 vector, derived from G207 by a 312 basepair deletion within the non-essential *α47* gene (Mavromara-Nazos et al., J. Virol. 60:807-812, 1986). Because of the overlapping transcripts encoding ICP47 and US11 in HSV, the deletion in α*47* also places the late *US11* gene under control of the immediate-early *α47* promoter, which enhances the growth properties of *γ34.5*^{*-*} mutants. An HSV-1 mutant designated hrR3, which is ribonucleotide reductace-defective, can also be used in the invention (Spear et al., Cancer Gene Ther. 7(7):1051-1059,2000).

The effects of the viruses used in the methods of the invention can be augmented if the virus also contains a heterologous nucleic acid sequence encoding one or more therapeutic products, for example, a cytotoxin, an immunomodulatory protein (i.e., a protein that either enhances or suppresses a host immune response to an antigen), a tumor antigen, an antisense RNA molecule, or a ribozyme. Examples of immunomodulatory proteins include, e.g., cytokines (e.g., interleukins, for example, any of interleukins 1-15, α, β, or γ-interferons, tumor necrosis factor, granulocyte macrophage colony stimulating factor (GM-CSF), macrophage colony stimulating factor (M-CSF), and granulocyte colony stimulating factor (G-CSF)), chemokines (e.g., neutrophil activating protein (NAP), macrophage chemoattractant and activating factor (MCAF), RANTES, and macrophage inflammatory peptides MIP-1a and MIP-1b), complement components and their receptors, immune system accessory molecules (e.g., B7.1 and B7.2), adhesion molecules (e.g., ICAM-1, 2, and 3), and adhesion receptor molecules. Examples of tumor antigens that can be produced as a result of using the present methods include, e.g., the E6 and E7 antigens of human papillomavirus, EBV-derived proteins (Van der Bruggen et al., Science 254:1643-1647, 1991), mucins (Livingston et al., Cur. Opin. Immun. 4(5):624-629, 1992), such as MUC1 (Burchell et al., Int. J. Cancer 44:691-696, 1989), melanoma tyrosinase, and MZ2-E (Van der Bruggen et al., *supra*). (Also see WO 94/16716 for a further description of modification of viral vectors to include genes encoding tumor antigens or cytokines.)

As is noted above, the heterologous therapeutic product can also be an RNA molecule, such as an antisense RNA molecule that, by hybridization interactions, can be used to block expression of a cellular or pathogen mRNA. Alternatively, the RNA molecule can be a ribozyme (e.g., a hammerhead or a hairpin-based ribozyme) designed either to repair a defective cellular RNA, or to destroy an undesired cellular or pathogen-encoded RNA (see, e.g., Sullenger, Chem. Biol. 2(5):249-253, 1995; Czubayko et al., Gene Ther. 4(9):943-949, 1997; Rossi, Ciba Found. Symp. 209:195-204, 1997; James et al., Blood 91(2):371-382, 1998; Sullenger, Cytokines Mol. Ther. 2(3):201-205, 1996; Hampel, Prog. Nucleic Acid Res. Mol. Bio. 58:1-39, 1998; Curcio et al., Pharmacol. Ther. 74(3):317-332, 1997).

A heterologous nucleic acid sequence can be inserted into a virus for use in the methods of the invention in a location that renders it under the control of a regulatory sequence of the virus. Alternatively, the heterologous nucleic acid sequence can be inserted as part of an expression cassette that includes regulatory elements, such as promoters or enhancers. Appropriate regulatory elements can be selected by one of ordinary skill in the art based on, for example, the desired tissue-specificity and level of expression. For example, a cell-type specific or tumor-specific promoter can be used to limit expression of a gene product to a specific cell type. This is particularly useful, for example, when a cytotoxic, immunomodulatory, or tumor antigenic gene product is being produced in a tumor cell in order to facilitate its destruction. In addition to using tissue-specific promoters, local administration of the viruss of the invention can result in localized expression and effect.

Examples of non-tissue specific promoters that can be used in the invention include the early Cytomegalovirus (CMV) promoter (U.S. Patent No. 4,168,062) and the Rous Sarcoma Virus promoter (Norton et al., Molec. Cell Biol. 5:281, 1985). Also, HSV promoters, such as HSV-1 IE and IE 4/5 promoters, can be used.

Examples of tissue-specific promoters that can be used in the invention include, for example, the prostate-specific antigen (PSA) promoter, which is specific for cells of the prostate; the desmin promoter, which is specific for muscle cells (Li et al., Gene 78:243, 1989; Li et al., J. Biol. Chem. 266:6562, 1991; Li et al., J. Biol. Chem. 268:10403, 1993); the enolase promoter, which is specific for neurons (Forss-Petter et al., J. Neuroscience Res. 16(1):141-156, 1986); the β-globin promoter, which is specific for erythroid cells (Townes et al., EMBO J. 4:1715,1985); the tau-globin promoter, which is also specific for erythroid cells (Brinster et al., Nature 283:499, 1980); the growth hormone promoter, which is specific for pituitary cells (Behringer et al., Genes Dev. 2:453, 1988); the insulin promoter, which is specific for pancreatic β cells (Selden et al., Nature 321:545, 1986); the glial fibrillary acidic protein promoter, which is specific for astrocytes (Brenner et al., J. Neurosci. 14:1030, 1994); the tyrosine hydroxylase promoter, which is specific for catecholaminergic neurons (Kim et al., J. Biol. Chem. 268:15689, 1993); the amyloid precursor protein promoter, which is specific for neurons (Salbaum et al., EMBO J. 7:2807, 1988); the dopamine β-hydroxylase promoter, which is specific for noradrenergic and adrenergic neurons (Hoyle et al., J. Neurosci. 14:2455, 1994); the tryptophan hydroxylase promoter, which is specific for serotonin/pineal gland cells (Boularand et al., J. Biol. Chem. 270:3757, 1995); the choline acetyltransferase promoter, which is specific for cholinergic neurons (Hersh et al., J. Neurochem. 61:306,1993); the aromatic L-amino acid decarboxylase (AADC) promoter, which is specific for catecholaminergic/5-HT/D-type cells (Thai et al., Mol. Brain Res. 17:227, 1993); the proenkephalin promoter, which is specific for neuronal/spermatogenic epididymal cells (Borsook et al., Mol. Endocrinol. 6:1502, 1992); the reg (pancreatic stone protein) promoter, which is specific for colon and rectal tumors, and pancreas and kidney cells (Watanabe et al., J. Biol. Chem. 265:7432, 1990); and the parathyroid hormone-related peptide (PTHrP) promoter, which is specific for liver and cecum tumors, and neurilemoma, kidney, pancreas, and adrenal cells (Campos et al., Mol. Rnfovtinol. 6:1642, 1992).

Examples of promoters that function specifically in tumor cells include the stromelysin 3 promoter, which is specific for breast cancer cells (Basset et al., Nature 348:699, 1990); the surfactant protein A promoter, which is specific for non-small cell lung cancer cells (Smith et al., Hum. Gene Ther. 5:29-35, 1994); the secretory leukoprotease inhibitor (SLPI) promoter, which is specific for SLPI-expressing carcinomas (Garver et al., Gene Ther. 1:46-50, 1994); the tyrosinase promoter, which is specific for melanoma cells (Vile et al., Gene Therapy 1:307, 1994; WO 94/16557; WO 93/GB 1730); the stress inducible grp78/BiP promoter, which is specific for fibrosarcoma/tumorigenic cells (Gazit et al., Cancer Res. 55(8):1660, 1995); the AP2 adipose enhancer, which is specific for adipocytes (Graves, J. Cell. Biochem. 49:219, 1992); the α-1 antitrypsin transthyretin promoter, which is specific for hepatocytes (Grayson et al., Science 239:786, 1988); the interleukin-10 promoter, which is specific for glioblastoma multiform cells (Nitta et al., Brain Res. 649:122, 1994); the c-erbB-2 promoter, which is specific for pancreatic, breast, gastric, ovarian, and non-small cell lung cells (Harris et al., Gene Ther. 1: 170, 1994); the α-B-crystallin/heat shock protein 27 promoter, which is specific for brain tumor cells (Aoyama et al., Int. J. Cancer 55:760, 1993); the basic fibroblast growth factor promoter, which is specific for glioma and meningioma cells (Shibata et al., Growth Fact. 4:277, 1991); the epidermal growth factor receptor promoter, which is specific for squamous cell carcinoma, glioma, and breast tumor cells (Ishii et al., Proc. Natl. Acad. Sci. U.S.A. 90:282, 1993); the mucin-like glycoprotein (DF3, MUC1) promoter, which is specific for breast carcinoma cells (Abe et al., Proc. Natl. Acad. Sci. U.S.A. 90:282, 1993); the mts1 promoter, which is specific for metastatic tumors (Tulchinsky et al., Proc. Natl. Acad. Sci. U.S.A. 89:9146, 1992); the NSE promoter, which is specific for small-cell lung cancer cells (Forss-Petter et al., Neuron 5:187,1990); the somatostatin receptor promoter, which is specific for small cell lung cancer cells (Bombardieri et al., Eur. J. Cancer 31A:184, 1995; Koh *et al*., Int. J. Cancer 60:843, 1995); the c-erbB-3 and c-erbB-2 promoters, which are specific for breast cancer cells (Quin et al., Histopathology 25:247, 1994); the c-erbB4 promoter, which is specific for breast and gastric cancer cells (Rajkumar et al., Breast Cancer Res. Trends 29:3, 1994); the thyroglobulin promoter, which is specific for thyroid carcinoma cells (Mariotti et al., J. Clin. Endocrinol. Meth. 80:468, 1995); the α-fetoprotein promoter, which is specific for hepatoma cells (Zuibel et al., J. Cell. Phys. 162:36, 1995); the villin promoter, which is specific for gastric cancer cells (Osborn et al., Virchows Arch. A. Pathol. Anat. Histopathol. 413:303, 1988); and the albumin promoter, which is specific for hepatoma cells (Huber, Proc. Natl. Acad. Sci. U.S.A. 88:8099, 1991).

The viruses can be administered by any conventional route used in medicine, either at the same time as an anticancer agent, as is described below, or shortly before or after anticancer agent administration. Also, the viruses can be administered by the same or a different route as the anticancer agent, as can be determined to be appropriate by those of skill in this art.

The viruses (or anticancer agents) used in the methods of the invention can be administered directly into a tissue in which an effect, e.g., cell killing and/or therapeutic gene expression, is desired, for example, by direct injection or by surgical methods (e.g., stereotactic injection into a brain tumor; Pellegrino et al., Methods in Psychobiology (Academic Press, New York, New York, 67-90, 1971)). An additional method that can be used to administer viruses into the brain is the convection method described by Bobo et al. (Proc. Natl. Acad. Sci. U.S.A. 91:2076-2080, 1994) and Morrison et al. (Am. J. Physiol. 266:292-305, 1994). In the case of tumor treatment, as an alternative to direct tumor injection, surgery can be carried out to remove the tumor, and the viruses inoculated into the resected tumor bed to ensure destruction of any remaining tumor cells. Alternatively, the viruses can be administered via a parenteral route, e.g., by an intravenous, intraarterial, intracerebroventricular, subcutaneous, intraperitoneal, intradermal, intraepidermal, or intramuscular route, or *via* a mucosal surface, e.g., an ocular, intranasal, pulmonary, oral, intestinal, rectal, vaginal, or urinary tract surface.

Any of a number of well-known formulations for introducing viral vectors into cells in mammals, such as humans, can be used in the invention. (See, e.g., *Remington's Pharmaceutical Sciences* (18^{th} edition), ed. A. Gennaro, 1990, Mack Publishing Co., Easton, PA.) However, the viruses can be simply diluted in a physiologically acceptable solution, such as sterile saline or sterile buffered saline, with or without an adjuvant or carrier.

The amount of vector to be administered depends, e.g., on the specific goal to be achieved, the strength of any promoter used in the vector, the condition of the mammal (e.g., human) intended for administration (e.g., the weight, age, and general health of the mammal), the mode of administration, and the type of formulation. In general, a therapeutically or prophylactically effective dose of, e.g., from about 10¹ to 10¹⁰ plaque forming units (pfu), for example, from about 5x10⁴ to 1x10⁶ pfu, e.g., from about 1x10⁵ to about 4x10⁵ pfu, although the most effective ranges may vary from host to host, as can readily be determined by one of skill in this art. Also, the administration can be achieved in a single dose or repeated at intervals, as determined to be appropriate by those of skill in this art.

### Anticancer Agents

Any of numerous anticancer agents (i.e., chemotherapeutic agents) can be used in the methods of the invention. These compounds fall into several different categories, including, for example, alkylating agents, antineoplastic antibiotics, antimetabolites, and natural source derivatives. Examples of alkylating agents that can be used in the invention include busulfan, caroplatin, carmustine, chlorambucil, cisplatin, cyclophosphamide (i.e., cytoxan), dacarbazine, ifosfamide, lomustine, mecholarethamine, melphalan, procarbazine, streptozocin, and thiotepa; examples of antineoplastic antibiotics include bleomycin, dactinomycin, daunorubicin, doxorubicin, idarubicin, mitomycin (e.g., mitomycin C), mitoxantrone, pentostatin, and plicamycin; examples of antimetabolites include fluorodeoxyuridine, cladribine, cytarabine, floxuridine, fludarabine, flurouracil (e.g., 5-fluorouracil (5FU)), gemcitabine, hydroxyurea, mercaptopurine, methotrexate, and thioguanine; and examples of natural source derivatives include docetaxel, etoposide, irinotecan, paclitaxel, teniposide, topotecan, vinblastine, vincristine, vinorelbine, taxol, prednisone, and tamoxifen. Additional examples of chemotherapeutic agents that can be used in the invention include asparaginase and mitotane.

Methods for administration of chemotherapeutic drugs are well known in the art and vary depending on, for example, the particular drug (or combination of drugs) selected, the cancer type and location, and other factors about the patient to be treated (e.g., the age, size, and general health of the patient). Any of the drugs listed above, or other chemotherapeutic drugs that are known in the art, are administered in conjunction with the mutant Herpes viruses described herein.

The virus and the anticancer agents can be administered, for example, on the same day, e.g., within 0-12 hours (e.g., within 1-8 or 2-6 hours) of one another, or can be administered on separate days, e.g., within 24, 48, or 72 hours, or within a week, of one another, in any order. In addition, they can be administered by the same or different routes, as can be determined to be appropriate by those of skill in this art (see, e.g., above). Specific examples of routes that can be used in the invention include intravenous infusion, the oral route, subcutaneous or intramuscular injection, as well as local administration, by use of catheters or surgery. The appropriate amount of drug to be administered can readily be determined by those of skill in this art and can range, for example, from 1 µg-10 mg/kg body weight, e.g., 10 µg-1 mg/kg body weight, 25 µg-0.5 mg/kg body weight, or 50 µg-0.25 mg/kg body weight. The drugs can be administered in any appropriate pharmaceutical carrier or diluent, such as physiological saline or in a slow-release formulation.

Examples of cancers can be treated using the methods of the invention, include cancers of nervous-system, for example, astrocytoma, oligodendroglioma, meningioma, neurofibroma, glioblastoma, ependymoma, Schwannoma, neurofibrosarcoma, neuroblastoma, pituitary tumors (e.g., pituitary adenoma), and medulloblastoma. Other types of cancers that can be treated using the methods of the invention, include, head and neck cancer, melanoma, prostate carcinoma, renal cell carcinoma, pancreatic cancer, breast cancer, lung cancer, colon cancer, gastric cancer, bladder cancer, liver cancer, bone cancer, fibrosarcoma, squamous cell carcinoma, neurectodermal, thyroid tumor, lymphoma (Hodgkin's and non-Hodgkin's lymphomas), hepatoma, mesothelioma, epidermoid carcinoma, cancers of the blood (e.g., leukemias), as well as other cancers mentioned herein.

### Experimental Results

The invention is based, in part, on the following experimental results, which show the synergistic activities of a mutant Herpes Virus (G207) and two anticancer agents, fluorodeoxyuridine (I) and Mitomycin C (II), in the treatment of cancer.

### I. Functional Interactions Between Fluorodeoxyuridine-induced Cellular Alterations and Replication of a Ribonucleotide Reductase-Negative Herpes Simplex Virus

As is noted above, G207 is an oncolytic herpes simplex virus (HSV), which is attenuated by inactivation of viral ribonucleotide reductase (RR) and deletion of both *γ*_{*1*}*34.5* genes. The cellular counterparts that can functionally substitute for viral RR and the carboxyl-terminal domain of ICP34.5 are cellular RR and the corresponding homologous domain of the growth arrest and DNA damage protein 34 (GADD34), respectively. Because the thymidylate synthetase (TS) inhibitor fluorodeoxyuridine (FUdR) can alter expression of cellular RR and GADD34, we examined the effect of FUdR on G207 bioactivity with the hypothesis that FUdR-induced cellular changes will alter viral proliferation and cytotoxicity. Replication of wild-type HSV-1 was impaired in presence of 10 nM FUdR whereas G207 demonstrated increased replication under the same conditions. Combined use of FUdR and G207 resulted in synergistic cytotoxicity. FUdR exposure caused elevation of RR activity at 10 nM and 100 nM whereas GADD34 was induced only at 100 nM. The effect of enhanced viral replication by FUdR was suppressed by hydroxyurea, a known inhibitor of RR. These results demonstrate that the growth advantage of G207 in FUdR-treated cells is primarily based on an RR-dependent mechanism. Although our findings show that TS inhibition impairs viral replication, the FUdR-induced RR elevation may overcome this disadvantage resulting in enhanced replication of G207. These data provide the cellular basis for the combined use of RR-negative HSV mutants and thymidylate synthetase inhibitors in the treatment of cancer. Our experimental results are described in further detail below.

### Materials and Methods

### Cell lines and culture

HCT8 cells with two different degrees of sensitivity to 5-fluorouracil (5-FU) and FUdR were used for this study. HCT8 cells were obtained from the American Type Culture Collection (CCL-224, Rockville, MD, USA). The resistant cell line was cloned from HCT8 cells after exposure to 15 µM 5-FU for 7 days (HCT8/FU7dR) as previously described (Aschele et al., Cancer Res. 52:1855-1864, 1992). Both cell lines were maintained in RPMI 1640 media supplemented with 10% fetal calf serum (FCS), 100 µg/ml penicillin, and 100 µg/ml streptomycin. Vero cells (African green monkey kidney) were grown in Eagle's minimal essential medium (MEM) supplemented with 10% FCS.

### Viruses

Creation of the multi-mutated, replication-competent type-1 herpes virus G207 has been described previously (Mineta et al., Nature Med. 1:983-943, 1995). G207 was constructed from the R3616 mutant based on wild-type HSV-1 strain F. This mutant contains a 1 kb deletion from the coding domains of both *γ*_{*1*}*34.5* loci and an insertion of the *Escherichia coli lacZ* gene into the *ICP6* gene which encodes the large subunit of ribonucleotide reductase. HSV-1(F) is the parental wild-type virus of G207, whereas KOS is wild-type HSV-1 of different strain. Viruses were propagated on Vero cells. G207 was a gift of S.D. Rabkin and R.L. Martuza. HSV-1 (F) and KOS were provided by MediGene, Inc. (Vancouver, Canada).

### p53 mutational analysis

Genomic DNA was extracted from HCT8 and HCT8/7dR cells. Exons 5 through 9 of the p53 gene were amplified by polymerase chain reaction and analyzed for mutations by single-strand confirmation polymorphism.

### Cytotoxicity assay

Cytotoxicity of G207 and FUdR (Floxuridine, Roche Laboratories Inc., Nutley, NJ) was assessed by measuring cytoplasmic lactate dehydrogenase (LDH) activity (CytoTox 96 non-radioactive cytotoxicity assay, Promega, Madison, WI). All cytotoxicity assays were performed in 24-well plates starting with 2 × 10⁴ cells per well. At various time points following start of treatment, adherent cells were washed with PBS and cytoplasmic LDH was released by lysis buffer (PBS, 1.2% v/v Triton X-100). Activity of the lysate was measured with a coupled enzymatic reaction, which converts a tetrazolium salt into a red formazan product. Absorbance was measured at 450 nm using a microplate reader (EL 312e, Bio-Tek Instruments, Winooski, VT). Cytotoxicicty was expressed as percentage of maximal LDH release of treated cells compared to untreated cells (control).

### Viral titration

Vero cultures were carried for at least one subculture in E-MEM, 2 mM L-glutamine, 10% FCS. Cultures were plated at a density of 1 x 10⁶ cells per well of 6-well plates and incubated at 37°C in 5% CO₂ in air in a humidified incubator. The following day, cultures were washed 2x with PBS, and serial dilutions of cell lysates (0.8 ml/well) were adsorbed onto triplicate dishes for 4 hours at 37°C. Cell lysates were prepared by 4 freeze-thaw cycles. Following adsorption, inoculum was removed, and cultures were overlaid with agar containing medium. Cultures were stained with neutral red 2 days post-inoculation, and plaque formation was assessed the next day.

### β-galactosidase activity

Activity of β-galactosidase was determined by monitoring the conversion of o-nitrophenyl galactoside (ONPG) to o-nitrophenol and galactose (β-Galactosidase Reporter Assay, Pierce, Rockford, IL). Cells were lysed and incubated with ONPG at 37°C for 30 min. The reaction rate was determined by spectrophotometric measurement of o-nitrophenol at λ=405 nm. Using the molar extinction coefficient for o-nitrophenol (ε_{λ} = 4.5 x 10³ M⁻¹ cm⁻¹), one unit of β-galactosidase activity was defmed as cleavage of 1 nmol ONPG to o-nitrophenol and galactose in 1 min at 37°C.

### Histochemical staining for β-galactosidase

Cells were trypsinized, resuspended in media, and washed with PBS. Cytospin slides were prepared by centrifuging 1 ml of a cell suspensions containing 1 × 10⁵ cells at 1000 rpm for 6 min. Slides were stained with X-gal (5-bromo-4-chloro-3-indolyl-β-D-galactopyranoside) and incubated for 4 hours at 37°C. After washing with PBS, slides were counterstained with 0.1% nuclear fast red.

### Cell cycle analysis

Cell cycle analysis was performed on nuclear preparations by flow cytometry as previously described (Nüsse et al., Cytometry 11:813-821, 1990). Briefly, cell monolayers were carefully washed with PBS to remove cellular debris. Following trypsinization, cells were washed in PBS and resuspended in NP40 solution (10 mM NaCl, 3.4 mM sodium citrate, 0.03% NP-40, 63 µM ethidium bromide, 10 µg/ml RNase A). After 1 hour incubation at room temperature, an equal volume of high-sucrose solution (0.25 M sucrose, 78 mM citric acid, 100 µM ethidium bromide) was added. DNA content of ethidium bromide-stained nuclei was determined on FACScalibur. Data were analyzed with FACStation running CellQuest software (Becton Dickinson, San Jose, CA).

### Cell extraction of ribonucleotide reductase

Cells grown in 250 cm²-flasks were trypsinized and washed twice in ice-cold PBS. Cells were centrifuged at 300 x g for 5 min at 4°C and resuspended in 3 volumes of Low Salt Extraction Buffer (10 mM HEPES, pH 7.2, 2 mM DTT). Viable cell count of the resuspension was determined by trypan blue exclusion. After 30 min incubation on ice, the cell suspension was drawn 10× through a 28G½ needle. The crude homogenate was centrifuged at 100,000 x g for 60 min at 4°C to remove cellular debris. The supernatant fraction was dialyzed against 1,000 volumes of the Low Salt Extraction Buffer for 4 hours with one buffer change after 2 hours using dialysis cassettes with a molecular weight cut-off of 10,000 (Slide-A-Lyzer Dialysis Cassettes, Pierce, Rockford, IL). The dialyzed extract was snap-frozen in liquid nitrogen and stored at -80°C until analysis. All extraction procedures were performed at 4°C.

### Assay for CDP reductase activity

Activity of ribonucleotide reductase was determined by a modified method of Steeper and Stuart (Steeper et al., Anal. Biochem. 34:123-130, 1970). Conversion of CDP to dCDP was monitored using [¹⁴C]CDP (53 mCi/mmol, Movarek Biochemicals, Inc.) as substrate and rattlesnake venom from Crotalus adamanteus (Sigma) to hydrolyze nucleotides to nucleosides. The reaction mixture contained the following concentrations of ingredients in a final volume of 150 µl: 40 µM CDP, 10 µM [¹⁴C]CDP (0.08 µCi), 6 mM DTT, 4 mM magnesium acetate, 2 mM ATP, 50 mM HEPES (pH 7.2), and 100 µl extract (0.2-0.7 mg protein). The enzyme reaction was carried out for 30 min at 37°C and stopped by incubation at 100°C for 4 min. Nucleotides were hydrolyzed by adding 50 µl carrier dCMP (6 mM dCMP, 2 mM MgCl₂, 6 mM Tris-HCl, pH 8.8) and 25 µl snake venom suspension (50 mg/ml). After 3 hours incubation at 37°C, the reaction mixture was heat-inactivated by boiling for 4 min. Heat-precipitated material was removed by centrifugation at 14,000 x g for 10 min at room temperature. [¹⁴C]deoxycytosine was separated from [¹⁴C]cytosine by covalent chromatography using phenylboronic acid-columns (BondElut PBA, Varian, Harbor City, CA). Triethenolamine Buffer (pH 10) was added to the supernatant fraction to a final concentration of 0.4 M and 1 ml of this mixture was applied to the column. Fractions were collected and measured for radioactivity by liquid scintillation spectrometry (LS 6000IC Liquid Scintillation System, Beckman Instruments, Inc., Fullerton, CA). One unit of enzyme activity was defined as conversion of 1 nmol CDP to the product dCDP in 1 hour at 37°C.

### Northern hybridization

Total cellular RNA was isolated by guanidine thiocyanate-phenol-chloroform extraction (Chomczynski et al., Anal. Biochem. 162:156-159, 1987). RNA was denatured, electrophoresed through a 1.2% formaldehyde-agarose gel, and blotted to a nitrocellulose membrane by standard techniques. Following prehybridization, membranes were hybridized in 50% formamide at 40°C to a full-length GADD34 and β-actin cDNA probe labeled with [³²P]dCTP by the random primer method. Membranes were washed and exposed to Hyperfilm (Amersham pharmacia biotek) at -80°C. Densitometric analysis was carried out on scanned films using the NIH image software. Relative GADD34 levels were calculated as the ratio GADD34/β-actin.

### Results

### Synergistic cytotoxicity of G207 and FUdR

HCT8 cells were more sensitive to FUdR compared to HCT8/7dR, as demonstrated by lower LDH release and a higher percentage of subG1 fraction (Figure 1A, Figures 5A and 5B). Both cell lines showed similar viral cytotoxicity profiles. Viral infection at a multiplicity of infection (MOI) of 1.0 or 0.1 resulted in complete cell kill at day 6 while G207 at an MOI of 0.01 had only marginal cytotoxic effects (Figures 1B and 1C). To test the hypothesis that FUdR can enhance viral cytotoxicity, we decided to use G207 at an MOI of 0.01 since viral cytotoxicity at MOI's of 1.0 and 0.1 was excessively high. G207 (MOI 0.01) combined with either 10 nM or 100 nM FUdR resulted in nearly complete kill of HCT8 cells by day 6 (Figure 1D). This effect was higher than the calculated additive effect from each single treatment indicating synergistic effects of combined treatment. Furthermore, degree of synergy was more pronounced in HCT8 cells than in the less FUdR-sensitive cell line HCT8/7dR (Figures 1D and 1E).

### Increased β-galactosidase expression in presence of FudR

To assess the effect of FUdR on viral infectivity, activity of β-galactosidase was measured as the product of the *lacZ* reporter gene in G207. Cells infected with G207 at an MOI of 0.01 followed by treatment with 10 nM FUdR showed the highest total expression of β-galactosidase at day 3 postinfection (pi), however, when normalized to viable cell count, exposure to 100 nM FUdR resulted in higher β-galactosidase activity than treatment with G207 only (Figures 2A and 2B). Histochemical staining for β-galactosidase of FUdR-exposed cells showed greater staining intensity and a higher proportion of cells positive for staining with X-gal (Figure 2C). The degree of enhanced infection by FUdR was more pronounced for HCT8 than for HCT8/7dR cells.

### Replication is enhanced for G207 but decreased for wild-type HSV-1 in the presence of FUdR

Single-step growth analysis demonstrated a 2-log higher viral yield for wild-type HSV (HSV-1(F), KOS) compared to G207 in HCT8 cells. Interestingly, burst size of G207 in the presence of FUdR was 3-fold higher at 36 hours pi than G207 alone, whereas replication of the parental wild-type virus HSV-1(F) was somewhat inhibited under the same conditions. We tested another wild-type HSV-1 (strain KOS) and found a similar reduction of replication in presence of 10 nM FUDR (Table 1). Multiple-step growth analysis revealed a significantly higher viral production in the presence of 10 nM FUdR in both cell lines compared to G207 infection alone. Peak titers and overall production of G207 were higher for the parental cell line HCT8 compared to HCT8/7dR cells (Figure 3).

### Effect of hydroxyurea on viral replication and β-galactosidase expression

The RR inhibitor hydroxyurea (HU) suppressed viral replication in HCT8 cells by 90%. Furthermore, HU was able to extinguish the FUdR-induced enhanced replication of G207. The degree of inhibition was the same for cells treated with HU alone (1.9 ± 0.5 ×10⁴ pfu) and for cells treated with HU and FUdR (2.1 ± 0.5 ×10⁴pfu). In contrast to viral production, neither FUdR nor HU had significant effects on β-galactosidase expression (Figure 4).

### Cell cycle alteration by FUdR

Exposure of asynchronously growing cells to FUdR resulted in an increase of S-phase fraction and a decrease of the G₁/G₀ fraction; however, this effect was dependent on drug concentration and cell line. Low FUdR concentrations of 10 nM increased S-phase fraction by 75% in HCT8 and 37% in HCT8/7dR by 24 hours. By 48 hours both cell lines showed a majority of cells in S-phase following treatment with 100 nM. In contrast to HCT8 cells, which were completely blocked at 100 nM FUdR at S-phase level, HCT8/7dR cells showed only a transient S-phase increase and were able to transit to an apparent G₂/M-phase. Additionally, we observed that 10-20% of HCT8/7dR cells undergo DNA endoreduplication in S-phase in the presence of 100 nM FUdR instead of moving to G₂/M. Accumulation of these cells in G₂/M may be due to there being 4N G₁ cells resulting from this endoreduplication (Figures 5A and 5B).

### Elevated activities of ribonucleotide reductase in the presence of FUdR

Since replication of G207 is dependent on cellular RR, we tested whether the thymidylate synthetase inhibitor FUdR has any effects on this cellular enzyme. Baseline activity of exponentially growing cells was approximately 3.2-fold higher for HCT8 cells compared to the chemoresistant cell line HCT8/7dR. Figure 6 shows the time-dependent course of RR activity during FUdR exposure. FUdR treatment resulted in an increase of RR activity in both cell lines. This increase was transient and peak activities were observed simultaneously with the FUdR-induced S-phase elevation at 24 hours following start of treatment (Figures 5A and 5B). The degree of activity induction was, however, more pronounced in HCT8 compared to HCTB/7dR cells. RR activity in HCT8 treated with 10 nM FUdR remained elevated following peak activity.

### Effect of FdUMP on the activity of ribonucleotide reductase

FdUMP is the active metabolite of FUdR and inhibits TS. Activity of mammalian RR is highly regulated by feedback inhibition of deoxynucleotides; we therefore tested the idea that FdUMP, the fluorinated form of dUMP, inhibits the activity of RR, which could interfere with replication of G207. Table 2 shows a dose-dependent decline of enzyme activity. Concentrations of FdUMP at 0.001 to 0.1 mM caused only a moderate inhibition of RR, with approximately 80 to 70% of the activity remaining. Substantial enzyme inhibition was measured in the presence of 1 mM FdUMP, a 10,000-fold higher concentration than the maximal FUdR concentration used in this study. *Expression of GADD34 in response to FUdR*

The GADD34 protein is expressed in response to DNA damage. GADD34 and the viral γ₁34.5 protein contain similar carboxyl-terminal domains that can functionally sustain protein synthesis under stress conditions. We therefore investigated whether FUdR as a DNA damaging agent can induce expression of GADD34 that can complement the *γ*_{*1*}*34.5* deletions in G207. FUdR at 100 nM induced GADD34 in both cell lines whereas 10 nM had almost no effect. When compared to untreated cells, densitometric reading revealed a 1.9- and 1.6-fold higher mRNA level at 24 and 48 hours, respectively for HCT8 and a 1.9-fold higher level at 48 hours for HCT8/7dR cells (Figure 7).

**Table 1:**

| Replication of wild-type HSV-1 and G207 in presence and absence of FUdR | | | |
|---|---|---|---|
| | Viral yield [10⁵ x pfu]^{a} | | |
| | HSV-1 (F) | KOS | G207 |
| Virus alone | 325 ± 25 | 119 ± 10 | 1.16 ± 0.22 |
| Virus + 10 nM FUdR | 215 ± 10 | 57 ± 2 | 3.42 ± 0.28 |
| Fold increase | 0.66 | 0.48 | 2.95 |

| | | | |
|---|---|---|---|
| ^{a} HCT8 cells were infected with HSV-1(F), KOS and G207 at an MOI of 2. After adsorption for 1 hour at 37°C, inoculum was removed, cells were washed with PBS, and medium containing 10 nM FUdR or control medium without FUdR was added. At 36 hours pi cells and supernatant were harvested and lysates were titrated on Vero cells by standard plaque assay. Data are presented as avg ± SEM of three independent determinations. | | | |

**Table 2:**

| Effect of FdUMP on ribonucleotide reductase activity | | | |
|---|---|---|---|
| FdUMP [mM] | RR activity ^{a,b} [nmol dCDP / h] | Activity [%] | Inhibition [%] |
| 0 | 0.61±0.05 | 100 | 0 |
| 0.001 | 0.47 ± 0.02 | 77.0 | 23.0 |
| 0.01 | 0.45 ± 0.02 | 73.8 | 26.2 |
| 0.1 | 0.43 ± 0.03 | 70.5 | 29.5 |
| 1 | 0.24 ± 0.02 | 39.3 | 60.7 |
| 10 | 0.14 ± 0.01 | 22.9 | 77.1 |

| | | | |
|---|---|---|---|
| ^{a} Ribonucleotide reductase was extracted from exponentially growing HCT8 cells as described under "Experimental Procedure". Extracts were incubated with FdUMP in cumulative concentrations and ribonucleotide reductase activity was measured. Data are presented as avg ± SEM of three independent determinations of ribonucleotide reductase activity. | | | |
| ^{b} 100 µl dialyzed cell extract contained 0.65 mg protein. | | | |

### II. Synergistic Anticancer Activity of Mitomycin C and a γ34.5 Deleted Oncolytic Herpes Virus (G207) is Mediated by Upregulation of GADD34

Oncolytic viruses used for gene therapy have been genetically modified to selectively target tumor cells while sparing normal host tissue. As is described above, the multimutant virus G207 has been attenuated by inactivation of viral ribonucleotide reductase and by deletion of both viral γ34.5 genes. Although G207 has effectively killed many tumor types in experimental models, it is well established that γ34.5 mutants exhibit markedly reduced antitumor efficacy when compared to viruses maintaining this gene. The mammalian homologue to the γ34.5 gene product is the GADD34 protein. This protein can functionally substitute for the γ34.5 gene and is also upregulated during DNA damage. The chemotherapy agent Mitomycin C was used in combination with G207 to upregulate GADD34 and to complement the γ34.5 gene deletion in an attempt to increase viral toxicity and antitumor efficacy. Using both the isobologram method and combination-index method of Chou-Talaley, significant synergism was demonstrated between Mitomycin C and G207 as treatment for gastric cancer both *in* *vitro* and *in vivo.* As a result of such synergism, a dose-reduction for each agent can be accomplished over a wide range of drug-effect levels without sacrificing tumor cell kill. As determined by Northern blot analysis, expression of GADD34 mRNA was increased by Mitomycin C treatment. These data indicate that Mitomycin C can be used to selectively restore the virulent phenotype of the γ34.5 gene in G207, and also provide a cellular basis for the combined use of DNA damaging agents and γ34.5 HSV mutants in the treatment of cancer. Our experimental results are described in further detail below.

### Materials and Methods

### Cell culture

The human gastric cancer cell line OCUM-2MD3 was obtained as a generous gift from Dr. Masakazu Yashiro at Osaka City University Medical School, Japan, and was maintained in DMEM HG supplemented with 2mM L-glutamine, 0.5 mM NaPyruvate, 10% fetal calf serum (FCS), 1% penicillin and 1% streptomycin. The human gastric cancer cell line MKN-45-P was obtained as a generous gift from Dr. Yutaka Yoneumura at Kanazawa University, Japan, and was maintained in RPMI supplemented with 10% FCS, 1% penicillin, and 1% streptomycin. The human lung cancer cell line A549 was obtained from the ATCC and maintained in F-12 supplemented with 10% FCS, 1% penicillin, and 1% streptomycin. Cells were all maintained in a 5% CO₂ humidified incubator.

### Virus

G207 is multi-mutated, replication-competent HSV constructed with deletions of both γ₁34.5 neurovirulence genes, and an *E.coli lacZ* insertion at U_{L}39, which codes for the large subunit of ribonucleotide reductase. The construction of G207 has been described elsewhere.

### Animals

Athymic nude mice 4-6 weeks old were used for all animal experiments. Animal studies were approved by the Memorial Sloan-Kettering Cancer Center Institutional Animal Care and Use Committee and performed under strict guidelines. Procedures were performed using methoxyflurane inhalation for anesthesia.

### Generation of peritoneally disseminated gastric cancer

An established murine xenograft model of gastric carcinomatosis was used as previously described (Bennett et al., Journal of Molecular Medicine 78:166-174, 2000; Yashiro et al., Clin. Exp. Metastasis 14:43-54, 1996). Intraperitoneal (i.p.) injection of 2x10⁶ OCUM-2MD3 cells reliably develops disseminated peritoneal tumor that seeds the omentum, small and large bowel mesentery, diaphragm, gonadal fat and hepatic hilum. Macroscopic nodules are present within three days after injection, and the development of overwhelming tumor burden, bloody ascites and cachexia occurs by four weeks post-injection. To assess tumor burden, animals were eviscerated at sacrifice and peritoneal tumor was stripped from associated abdominal organs as previously described . Tumor burden was then assessed by weight.

### In vitro cytotoxicity of MMC and G207

Cytotoxicity assays were performed by plating 1x10⁴ cells/well into 96 well assay plates (Costar, Corning Inc., Coming, NY). MKN-45-P and OCUM-2MD3 cells were treated with either media alone (control wells), Mitomycin C alone (Bristol Laboratories, Princeton, NJ), G207 alone, or combination therapy using both G207 and MMC. Combination therapy was performed using serial dilutions of MMC and G207 in a 1:10 ratio for the OCUM-2MD3 cell line, and a 1:25 ratio for the MKN-45-P cell line. These ratios were determined by estimating the ED50 for each drug in initial experiments and by using these doses to determine the ratio of combination therapy. Percent cell survival for each group (vs. controls) was calculated 5d after treatment using a standard MTT (3-(4,5-dimethylthiazol-2-yl)-2,5-diphenyl-2H tetrazolium bromide) bioassay. A micro-plate reader was used to evaluate for the presence of the MTT-formazan product at 550 nm. Background optical density (OD) was subtracted from the OD readings of all samples. Cell viability was then calculated by dividing the mean absorbance (OD)(n=6) of the treated wells by the mean absorbance (OD)(n=6) of the untreated (control) wells.

### Pharmacologic analysis of synergy between MMC and G207

The multiple drug effect analysis of Chou and Talalay was used to determine the pharmacologic interaction between G207 and MMC. This method defines the expected additive effect of two agents and then quantifies synergism or antagonism by determining how much the combination effect differs from the expected additive effect. The equations and computer software used for data analysis have been described in detail elsewhere (Chou, T., editors. Academic Press, New York. 61-102,1991; Chou et al., Advances in Enzyme Regulation 22:27-55, 1984; Chou et al., Manual and Software for IBM-PC 1987; Chou, J., Academic Press, New York 223-244, 1991). The combination index equation is used to precisely analyze two-drug combinations. Interpretation of CI values are defined such that a CI=1 indicates an additive effect, while a CI<1 and a CI>1 indicate synergism and antagonism, respectively. Cytotoxicity data obtained from the experiments described above were used in the Chou-Talalay analysis. These data generated CI values for each dose and corresponding effect level, referred to as the fraction affected (Fa). Based on the actual experimental data, computer software was used to calculate serial CI values over an entire range of effect levels (Fa) from 5-95%. These data were then used to generate Fa-CI plots, which is an effect-oriented means of presenting the data. Data were also analyzed by the isobologram technique, which is dose-oriented. The axes on an isobologram represent the doses of each drug. For any given Fa value, two points on the *x* and *y* axes are chosen that correspond to the doses of each drug necessary to generate that given Fa value. The straight line drawn between these two points corresponds to the possible combination doses that would be required to generate the same Fa value, assuming that the interaction between the two drugs is strictly additive. The observed experimental concentrations actually required to achieve a given Fa value are then added to the plot. If these points lie on the straight line then the effect is additive at that Fa value. If the point lies to the left of the straight line then the effect is synergistic, and if the point lies to the right of the straight line then the effect is antagonistic at that Fa value. Another calculation available using the combination index method is the dose-reduction index (DRI). The DRI is a determination of the fold-dose reduction allowed for each drug when given in synergistic combination, as compared to the concentration of single agent therapy needed to achieve the same effect level. A DRI>1 signifies a favorable reduction in toxicity while still maintaining therapeutic efficacy.

### In vitro viral growth analysis

The ability of G207 to replicate within OCUM-2MD3cells in the presence of MMC was evaluated by viral growth analysis. 1.5x10⁵ OCUM-2MD3 cells/well were plated into 6-well plates (Costar, Corning Inc., Corning, NY). Cells were then infected with either G207 at an MOI=0.01 alone, or with G207 at an MOI=0.01 in combination with MMC at 0.01 µg/cc, 0.02 µg/cc, or 0.04 µg/cc. Cells and media were harvested at 0h, 24h, 48h, 72h, and 120h post-infection. After three cycles of freeze-thaw lysis, standard plaque assay was performed on Vero cells to evaluate viral titers. All samples were performed in triplicate.

### Northern Hybridization Analysis for GADD34 in cells treated with MMC in vitro

Cells were cultured with 12cc culture media containing 0 µg/ml (untreated), 0.005 µg/ml (low dose), or 0.04 µg/ml (high dose) of Mitomycin-C (Bristol Laboratories, Princeton, NJ). Cells were harvested by trypsinization at 24 and 48 hours. Total RNA was prepared using a total RNA isolation system (Promega, Madison, WI) and RNA content was measured by optical density at 260 nm. 7 µg of RNA per sample was loaded in a denaturing 1.2% agarose gel. Electrophoretic separation, RNA transfer to a nitrocellulose membrane (Intergen, Purchase, NY), hybridization (50% formamide at 40°C), and autoradiographic identification were done by standard techniques. The cDNA clone GADD34 containing a 2.4 kb insert was provided by Dr.A. Fornace, Jr, and the cDNA clone α-actin containing a 1.1 kb insert was acquired from ATCC (Manassas, VA)(Hollander et al., J. Biol. Chem. 272:13731-13737, 1997). cDNA that had been excised from plasmid vectors was labelled with [³²P]dCTP by the random-primer labelling method (Stratagene, La Jolla, CA).

### Treatment of gastric carcinomatosis with intraperitoneal G207 and MMC

The ability of G207 and MMC to reduce tumor burden *in vivo* was evaluated in a model of gastric carcinomatosis. Animals were all injected i.p. with 2x10⁶ OCUM-2MD3 cells and treated 3d later. Experimental groups (n=7) were treated by intraperitoneal injection of serum free media (controls), 1x10⁶ pfu of G207, 5x10⁶ pfu of G207, 0.1 mg/kg MMC, or as combination therapy using 0.1 mg/kg MMC with either 1x10⁶ or 5x10⁶ pfu of G207. Animals were sacrificed 4 weeks later and tumor burden was assessed as described (Bennett et al., Journal of Molecular Medicine 78:166-174, 2000).

### Results

### In vitro cytotoxicity of MMC and G207

Both G207 and MMC demonstrate dose-dependent cytotoxicity against OCUM-2MD3 and MKN-45-P gastric cancer cells. Combination therapy killed more tumor cells than either single agent alone and showed greater efficacy than the expected additive effect. Data are presented as mean (±SEM) cell survival vs. controls for OCUM-2MD3 cells (Figure 8A) and for MKN-45-P cells (Figure 8B).

### Pharmacologic analysis of synergy between MMC and G207

Two methods were employed to determine synergy between G207 and MMC, the combination-index method and the isobologram method. Chou-Talaley analysis demonstrated that the CI values remained <1 over the entire range of Fa values for both the OCUM-2MD3 (Figure 9A) and MKN-45-P (Figure 9B) cell lines. Moderate synergism was demonstrated for the OCUM-2MD3 cell line, while strong synergism was demonstrated for the MKN-45-P cell line. The dose-reduction index (DRI) was calculated for each Fa value. For the OCUM-2MD3 cell line, both MMC and G207 doses could be lowered 2-3 fold when given as combination therapy (Table 3). For the MKN-45-P cell line, MMC doses could be lowered 2-9 fold and G207 doses could be lowered 2-4 fold when given as combination therapy (Table 4). DRI values >1 indicate that a reduction in toxicity can be achieved without loss of efficacy. Isobolograms were constructed for the doses of MMC and G207 necessary to kill 90% of cells (ED90), 70% of cells (ED70) and 50% of cells (ED50) (Figures 10A and 10B). Experimental combination data points were at drug and viral concentrations well below the expected additive effect line for each of these Fa values (0.5, 0.7, and 0.9). These studies both confirmed synergism between MMC and G207 for both cell lines.

### In vitro viral growth analysis

Replication of G207 in OCUM-2MD3 cells demonstrated a decline in viral yield in the presence of higher doses of MMC. A 155-fold increase in viral titers was observed 5d after infecting OCUM-2MD3 cells with G207. In the presence of 0.01 µg/cc MMC, a 24-fold increase in viral titers was observed over 5d post-infection. In the presence of 0.02 and 0.04 µg/cc MMC, there was an 8-fold and 2-fold increase in viral yields, respectively. Lower viral yields measured with combination chemotherapy may be secondary to significant loss of cellular substrate, especially given the synergistic cytotoxicity of combination therapy.

### Northern Hybridization Analysis for GADD34 in cells treated with MMC in vitro

RNA extracted from cells that were not treated with MMC served as negative controls (lane 1), while positive controls (lane 6) demonstrated the expected GADD34 band at 2.4 kb (Figure 11). In all conditions, an approximately equal amount of the cellular α-actin gene was expressed. OCUM cells harvested 24 hours (lane 2) after treatment with low dose Mitomycin C (0.005 µg/cc) did not show any band at the expected size, while cells treated with high dose MMC (0.04 µg/cc) demonstrated a significant 2.4 kb band (lane 3). At 24 hours after high dose treatment a 2.49 fold increase in the intensity of the GADD34 band was measured when compared to the negative control. OCUM cells harvested 48 hours after treatment with low dose MMC did not show any GADD34 band (lane 4), while high dose therapy showed a discrete band at 2.4 kb (lane 5)(Figure 11). At 48 hours after high dose treatment, a 3.21 fold increase in intensity was noted (Figure 11).

### Treatment of gastric carcinomatosis with intraperitoneal G207 and MMC

Mice with gastric carcinomatosis were treated intraperitoneally with G207, MMC, or a combination of these agents. Efficacy of therapy was evaluated by weighing peritoneal tumor burden from mice at the time of sacrifice. Mean peritoneal tumor burden (±SEM) was 2470 (±330) mg for control mice, 1210 (±300) mg for mice treated with 1x10⁶ pfu of G207 (P=0.02 vs. controls), and 1490 (±310) mg for mice treated with 0.1 mg/kg MMC (P=0.06 vs. controls)(Figure 12). Combination therapy using 1x10⁶ pfu of G207 and 0.1 mg/kg MMC resulted in a mean tumor burden of 350 (±150) mg (P<0.001 vs. controls), which was statistically different from 1x10⁶ pfu of G207 alone (P=0.03) and from MMC therapy alone (P=0.01)(Figure 5). Viral therapy with 5x10⁶ pfu of G207 resulted in a mean tumor burden of 990 (±320) mg (P<0.01 vs. controls)(data not shown). Combination therapy using 5x10⁶ pfu of G207 and 0.1 mg/kg MMC resulted in a mean tumor burden of 100 (±60) mg (P<0.01.vs. controls), which was statistically different from 5x10⁶ pfu of G207 alone (P=0.04) and from MMC therapy alone (P<0.01).

**Table 3:**

| Drug and viral doses needed to kill various fractions (Fa) of OCUM-2MD3 cells, and fold-dose reduction possible when agents are delivered in combination. | | | | |
|---|---|---|---|---|
| *Fraction affected (Fa)* | *MMC alone (ug*/*cc)* | *G207 alone (MOI)* | *MMC dose reduction index* | *G207 dose reduction index* |
| 10% | 0.009 | 0.08 | 3.6 | 3.0 |
| 20% | 0.014 | 0.11 | 3.5 | 2.8 |
| 30% | 0.017 | 0.14 | 3.4 | 2.7 |
| 40% | 0.021 | 0.17 | 3.3 | 2.6 |
| 50% | 0.026 | 0.20 | 3.3 | 2.6 |
| 60% | 0.031 | 0.24 | 3.2 | 2.5 |
| 70% | 0.038 | 0.29 | 3.2 | 2.5 |
| 80% | 0.048 | 0.37 | 3.1 | 2.4 |
| 90% | 0.070 | 0.53 | 3.0 | 2.3 |
| 95% | 0.100 | 0.74 | 2.9 | 2.2 |

**Table 4:**

| Drug and viral doses needed to kill various fractions (Fa) of MKN-45-P cells, and fold-dose reduction possible when agents are delivered in combination. | | | | |
|---|---|---|---|---|
| *Fraction affected (Fa)* | *MMC alone (ug*/*cc)* | *G207 alone (MOI)* | *MMC dose reduction index* | *G207 dose reduction index* |
| 10% | 0.014 | 0.34 | 2.4 | 2.3 |
| 20% | 0.027 | 0.57 | 3.0 | 2.6 |
| 30% | 0.040 | 0.79 | 3.5 | 2.7 |
| 40% | 0.056 | 1.05 | 3.9 | 2.9 |
| 50% | 0.077 | 1.35 | 4.3 | 3.0 |
| 60% | 0.105 | 1.73 | 4.8 | 3.2 |
| 70% | 0.147 | 2.28 | 5.4 | 3.3 |
| 80% | 0.222 | 3.19 | 6.2 | 3.5 |
| 90% | 0.413 | 5.28 | 7.6 | 3.9 |
| 95% | 0.732 | 8.41 | 9.2 | 4.2 |

All references cited above are incorporated by reference in their entirety. Other embodiments are within the following
1. A method of treating cancer in a patient, said method comprising administering to said patient (i) an attenuated herpes virus in which a γ34.5 gene is inactivated, and (ii) a chemotherapeutic drug.
2. The method of 1, wherein a ribonucleotide reductase gene is inactivated in said attenuated herpes virus.
3. The method of 1, wherein two γ34.5 genes are inactivated in said attenuated herpes virus.
4. The method of 1, wherein said attenuated herpes virus is G207.
5. The method of 1, wherein said chemotherapeutic drug is an alkylating agent.
6. The method of 5, wherein said alkylating agent is selected from the group consisting of busulfan, caroplatin, carmustine, chlorambucil, cisplatin, cyclophosphamide, dacarbazine, ifosfamide, lomustine, mecholarethamine, melphalan, procarbazine, streptozocin, and thiotepa.
7. The method of 1, wherein said chemotherapeutic drug is an antineoplastic antibiotic.
8. The method of 7, wherein said antineoplastic antibiotic is selected from the group consisting of bleomycin, dactinomycin, daunorubicin, doxorubicin, idarubicin, mitomycin, mitoxantrone, pentostatin, and plicamycin.
9. The method of 8, wherein antineoplastic antibiotic is mitomycin C.
10. The method of 1, wherein said chemotherapeutic drug is an antimetabolite.
11. The method of 10, wherein said antimetabolite is selected from the group consisting of thymidylate synthetase inhibitors, cladribine, cytarabine, floxuridine, fludarabine, flurouracil, gemcitabine, hydroxyurea, mercaptopurine, methotrexate, and thioguanine.
12. The method of 11, wherein said thymidylate synthetase inhibitor is fluorodeoxyuridine.
13. The method of 1, wherein said cancer is selected from the group consisting of astrocytoma, oligodendroglioma, meningioma, neurofibroma, glioblastoma, ependymoma, Schwannoma, neurofibrosarcoma, neuroblastoma, pituitary adenoma, medulloblastoma, head and neck cancer, melanoma, prostate carcinoma, renal cell carcinoma, pancreatic cancer, breast cancer, lung cancer, colon cancer, gastric cancer, bladder cancer, liver cancer, bone cancer, fibrosarcoma, squamous cell carcinoma, neurectodermal, thyroid tumor, Hodgkin's lymphoma, non-Hodgkin's lymphoma, hepatoma, mesothelioma, epidermoid carcinoma, and cancers of the blood.
14. The method of 1, wherein said herpes virus comprises a gene encoding a heterologous gene product.
15. The method of 14, wherein said heterologous gene product comprises a vaccine antigen.
16. The method of 14, wherein said heterologous gene product comprises an immunomodulatory protein.
17. A method of treating cancer in a patient, said method comprising administering to said patient (i) an attenuated herpes virus in which a ribonucleotide reductase gene is inactivated and (ii) a chemotherapeutic drug.
18. The method of 17, wherein said chemotherapeutic drug is an alkylating agent.
19. The method of 18, wherein said alkylating agent is selected from the group consisting of busulfan, caroplatin, carmustine, chlorambucil, cisplatin, cyclophosphamide, dacarbazine, ifosfamide, lomustine, mecholarethamine, melphalan, procarbazine, streptozocin, and thiotepa.
20. The method of 17, wherein said chemotherapeutic drug is an antineoplastic antibiotic.
21. The method of 20, wherein said antineoplastic antibiotic is selected from the group consisting of bleomycin, dactinomycin, daunorubicin, doxorubicin, idarubicin, mitomycin, mitoxantrone, pentostatin, and plicamycin.
22. The method of 21, wherein antineoplastic antibiotic is mitomycin C.
23. The method of 17, wherein said chemotherapeutic drug is an antimetabolite.
24. The method of 23, wherein said antimetabolite is selected from the group consisting of thymidylate synthetase inhibitors, cladribine, cytarabine, floxuridine, fludarabine, flurouracil, gemcitabine, hydroxyurea, mercaptopurine, methotrexate, and thioguanine.
25. The method of 24, wherein said thymidylate synthetase inhibitor is fluorodeoxyuridine.
26. The method of 17, wherein said cancer is selected from the group consisting of astrocytoma, oligodendroglioma, meningioma, neurofibroma, glioblastoma, ependymoma, Schwannoma, neurofibrosarcoma, neuroblastoma, pituitary adenoma, medulloblastoma, head and neck cancer, melanoma, prostate carcinoma, renal cell carcinoma, pancreatic cancer, breast cancer, lung cancer, colon cancer, gastric cancer, bladder cancer, liver cancer, bone cancer, fibrosarcoma, squamous cell carcinoma, neurectodermal, thyroid tumor, Hodgkin's lymphoma, non-Hodgkin's lymphoma, hepatoma, mesothelioma, epidermoid carcinoma, and cancers of the blood.
27. The method of 17, wherein said herpes virus comprises a gene encoding a heterologous gene product.
28. The method of 27, wherein said heterologous gene product comprises a vaccine antigen.
29. The method of 27, wherein said heterologous gene product comprises an immunomodulatory protein.

## Claims

1. Use of
(i) an attenuated mutant herpes virus, and
(ii) irinotecan
for the preparation of a pharmaceutical composition for treating cancer.

2. The use of claim 1, wherein in the attenuated mutant herpes virus a γ34.5 gene is inactivated.

3. The use of claim 1 or 2, wherein a ribonucleotide reductase gene is inactivated in said attenuated mutant herpes virus.

4. The use of any of claims 1 to 3, wherein two γ34.5 genes are inactivated in said attenuated mutant herpes virus.

5. The use of claim 4, wherein said attenuated mutant herpes virus is G207.

6. The use of claim 1, wherein in the attenuated mutant herpes virus a ribonucleotide reductase gene is inactivated.

7. The use of any of claims 1 to 6, wherein said cancer is selected from the group consisting of astrocytoma, oligodendroglioma, meningioma, neurofibroma, glioblastoma, ependymoma, Schwannoma, neurofibrosarcoma, neuroblastoma, pituitary adenoma, medulloblastoma, head and neck cancer, melanoma, prostate carcinoma, renal cell carcinoma, pancreatic cancer, breast cancer, lung cancer, colon cancer, gastric cancer, bladder cancer, liver cancer, bone cancer, fibrosarcoma, squamous cell carcinoma, neurectodermal, thyroid tumor, Hodgkin's lymphoma, non-Hodgkin's lymphoma, hepatoma, mesothelioma, epidermoid carcinoma, and cancers of the blood.

8. The use of any of claims 1 to 7, wherein said herpes virus comprises a gene encoding a heterologous gene product.

9. The use of claim 8, wherein said heterologous gene product comprises a vaccine antigen.

10. The use of claim 8, wherein said heterologous gene product comprises an immunomodulatory protein.
